# EUROPEAN PATENT SPECIFICATION

(11) **EP 2 027 821 B1**
(45) Date of publication and mention of the grant of the patent: **31.12.2014**
(21) Application number: 08014863.8
(22) Date of filing: 21.08.2008
(51) Int. Cl.: A61B 17/29, A61B 17/32, A61B 18/14

(54) **Surgical operating apparatus**
Chirurgische Operationsvorrichtung
Appareil pour opération chirurgicale

(30) Priority: 24.08.2007 US 844504
(43) Date of publication of application: 25.02.2009
(73) Proprietor: Olympus Medical Systems Corp., Tokyo 151-0072 (JP)
(72) Inventor: Chie, Yachi, Hachioji-shi Tokyo 192-8512 (JP)
(74) Representative: von Hellfeld, Axel

(56) References cited:
- WO-A2-2006/042210
- WO-A2-2007/143439
- US-A1- 2005 033 337

## Description

The present invention relates to a surgical operating apparatus in which a hand switch is disposed in an operation portion of a surgical instrument.

Surgical operating apparatuses generally include a surgical instrument such as a pair of forceps. For example, in Jpn. Pat. Appln. KOKAI Publication No. 9-327465 (Patent document 1), a treatment portion is provided at the distal end of an insertion portion to be inserted into a body, and an operation portion for operating the treatment portion is provided at the proximal end of the insertion portion. This surgical instrument has an openable/closable handle in the operation portion. One handle switch is attached to this handle. The handle switch is configured to be operated by the finger of a user gripping the handle during the use of this surgical instrument.

Furthermore, Jpn. Pat. Appln. KOKAI Publication No. 2003-126116 (Patent document 2) has disclosed a configuration in which two switches are provided in the vicinity of levers of two handles disposed in an operation portion of a surgical instrument.

There has heretofore been a possibility in the surgical instrument that it is difficult to distinguish among a plurality of switches depending on the position of the finger when the switches are operated with the index finger of the user gripping the handle.

Moreover, there is a problem of extreme fatigue from the switch operation when the switches are positioned immediately above the middle finger. There is also a problem of the movement of the position of the treatment portion at the distal end of the surgical instrument due to the movement of other fingers following the movement of the index finger when the switches are operated with the index finger.

WO 2006/042210 A2 discloses a coagulator for cutting, coagulating and clamping of tissue during surgical procedures according to the preamble of claim 1. The coagulator comprises an operation portion with a clamping mechanism which may be activated by thumb and middle finger of an operator cooperating with handles. Switches may be actuated by an index finger of the operator. The switches are separated from each other by a bulging portion.

US 2005/033337 A1 discloses a coagulator comprising an ultrasonic generator activated by switches. The switches are placed on a handle for being selected by the index finger of an operator. The handle could be considered as being an operation portion. In order to prevent an accidental selection of one of the switches, the latter are spaced by an appropriate distance. A clamp arm is operated by a thumb squeeze trigger. The remaining fingers of the operator rest on the handle.

WO 2007/143439 A2, which has been published after the priority date of the present document, discloses an ultrasonic coagulator for cutting, coagulating and clamping of tissue during surgical procedures. The coagulator comprises a housing that could be considered to be comparable to an operation portion. The coagulator also comprises a clamping mechanism which may be activated by a trigger handle for insertion of a thumb of an operator. A further fixed handle permits the insertion of the ring finger of the operator and permits the pinkie of the operator to rest externally. Two switches, which are separated from each other by a bulging portion, are actuated by an index and a middle finger of the operator.

The present invention has been made in view of the foregoing circumstances, and is directed to provide a surgical operating apparatus, wherein a plurality of switches having different functions can be easily distinguished from each other, the switches are easily operated with the index finger of a user gripping a handle, fatigue is lessened, and the position of a treatment portion at the distal end of a surgical instrument can be prevented from moving during the operation of the switch.

A surgical operating apparatus in one aspect of the present invention comprises: an insertion portion which has a distal end and a proximal end and which has a long axis and which is inserted into a body; a treatment portion which is disposed at the distal end of the insertion portion and which has a plurality of selectable surgical functions; an operation portion disposed at the proximal end of the insertion portion; a plurality of switches which are provided in the operation portion and which select the surgical functions; and a bulging portion which is disposed between the switches and which divides the switches and which doubles as a finger receiving portion.

The operation portion has, on a front side thereof, a switch attachment surface onto which the plurality of switches are attached, the plurality of switches are arranged in a vertical direction of the switch attachment surface, and the bulging portion has an extension which continuously extends from the switch attachment surface of the operation portion over a least one of the lateral sides thereof.

Preferably, the bulging portion is set so that the height of projection of this bulging portion from the switch attachment surface is larger than the height of projection of the plurality of switches from the attachment surface.

Preferably, the operation portion has a main body of this operation portion and two handle elements to operate the treatment portion, the two handle elements has a fixed handle element fixed to the main body of the operation portion and extending on a lateral side of the long axis, and a movable handle element supported to be openable/closable with respect to the fixed handle element, and the attachment surface is provided at a junction between the main body of the operation portion and the fixed handle element.

Preferably, the movable handle element has a thumb insertion ring portion into which a thumb is inserted, the fixed handle element has a multiple finger insertion ring portion into which a plurality of fingers except for the thumb and index finger are inserted, the switch attachment surface has a curving surface curving along a flow line on which the index finger moves in a condition where the thumb is inserted into the thumb insertion ring portion and the plurality of fingers except for the thumb and index finger are inserted into the multiple finger insertion ring portion.

Preferably, the operation portion is set so that an angle [alpha] between a tangent line of a front surface of the multiple finger insertion ring portion of the fixed handle element and a tangent line of a front surface of the switch attachment surface is larger than 90°.

Preferably, the main body of the operation portion has a switch unit in which two switches are integrated into one unit, and a concave unit receiver to which the switch unit is attached, the switch unit has push buttons for the two switches, a flexible wiring line circuit board for the two switches, and a flexible base member in which the wiring line circuit board is embedded in insulating elastic members, and the base member is attached to the unit receiver so that this base member curves along the curving surface.

Preferably, the unit receiver has, in parts corresponding to the push buttons for the two switches, boss portions which receive force to push the push buttons for the two switches.

Preferably, the two switches have a first switch which is disposed on the upper side of the switch attachment surface and which selects a frequently used first surgical function of the plurality of surgical functions, and a second switch which is disposed on the lower side of the switch attachment surface and which selects another second surgical function of the plurality of surgical functions.

Preferably, the first surgical function is a function to simultaneously output an ultrasonic treatment output and a high-frequency treatment output, and the second surgical function is a function to independently output the high-frequency treatment output alone.

Preferably, the first surgical function is a function to output an ultrasonic treatment output in a maximum output state, and the second surgical function is a function to output an ultrasonic treatment output in a preset arbitrary set output state lower than the maximum output state.

Preferably, a finger pad portion formed of an elastic material is detachably attached to at least one of the thumb insertion ring portion and the multiple finger insertion ring portion.

Preferably, the movable handle element has a finger hook in an upper portion of the thumb insertion ring portion.

The invention can be more fully understood from the following detailed description when taken in conjunction with the accompanying drawings, in which:
FIG. 1 is a perspective view showing the schematic configuration of the whole ultrasonic treatment apparatus in a first embodiment of the present invention;
FIG. 2 is a perspective view showing how continuous parts of the ultrasonic treatment apparatus in the first embodiment are detached;
FIG. 3A is a plan view showing the distal end of a sheath unit of the ultrasonic treatment apparatus in the first embodiment;
FIG. 3B is a plan view showing the distal end of a probe unit of the ultrasonic treatment apparatus in the first embodiment;
FIG. 4A is a longitudinal sectional view showing the distal end of the sheath unit of the ultrasonic treatment apparatus in the first embodiment;
FIG. 4B is a longitudinal sectional view showing an insulating coating on the inner peripheral surface of an inner cylinder;
FIG. 5 is a sectional view along the V-V line in FIG. 4A;
FIG. 6 is a sectional view along the VI-VI line in FIG. 4A;
FIG. 7 is a sectional view along the VII-VII line in FIG. 4A;
FIG. 8 is a longitudinal sectional view showing the proximal end of the sheath unit of the ultrasonic treatment apparatus in the first embodiment;
FIG. 9A is a sectional view along the IXA-IXA line in FIG. 8;
FIG. 9B is a sectional view along the IXB-IXB line in FIG. 8;
FIG. 10 is a sectional view along the X-X line in FIG. 8;
FIG. 11 is a sectional view along the XI-XI line in FIG. 8;
FIG. 12 is a perspective view showing a connecting pipe member of the sheath unit of the ultrasonic treatment apparatus in the first embodiment;
FIG. 13 is a side view showing the connecting pipe member of the sheath unit of the ultrasonic treatment apparatus in the first embodiment;
FIG. 14 is a side view showing how a handle unit and a transducer unit of the ultrasonic treatment apparatus in the first embodiment are coupled to each other;
FIG. 15 is a longitudinal sectional view showing a unit coupling part of the ultrasonic treatment apparatus in the first embodiment;
FIG. 16A is a longitudinal sectional view showing the internal configuration of the handle unit of the ultrasonic treatment apparatus in the first embodiment;
FIG. 16B is a longitudinal sectional view showing the internal configuration wherein a switch unit is detached from the handle unit of the ultrasonic treatment apparatus in the first embodiment;
FIG. 17A is a sectional view along the 17-17 line in FIG. 15 showing a state before the engagement between the handle unit and the sheath unit of the ultrasonic treatment apparatus in the first embodiment;
FIG. 17B is a sectional view along the 17-17 line in FIG. 15 showing a state after the engagement between the handle unit and the sheath unit of the ultrasonic treatment apparatus in the first embodiment;
FIG. 18 is a sectional view along the 18-18 line in FIG. 15;
FIG. 19 is a sectional view along the 19-19 line in FIG. 15;
FIG. 20 is a sectional view along the 20-20 line in FIG. 15;
FIG. 21 is a sectional view along the 21-21 line in FIG. 15;
FIG. 22 is a sectional view along the 22-22 line in FIG. 15;
FIG. 23 is a sectional view along the 23-23 line in FIG. 15;
FIG. 24 is a sectional view along the 24-24 line in FIG. 15;
FIG. 25 is a sectional view along the 25-25 line in FIG. 15;
FIG. 26 is a perspective view showing an electrode holding member of the ultrasonic treatment apparatus in the first embodiment;
FIG. 27 is a front view showing the electrode holding member of the ultrasonic treatment apparatus in the first embodiment;
FIG. 28 is a side view showing the electrode holding member of the ultrasonic treatment apparatus in the first embodiment;
FIG. 29 is a perspective view showing an electrode member of the ultrasonic treatment apparatus in the first embodiment;
FIG. 30 is a transverse sectional view showing the electrode member of the ultrasonic treatment apparatus in the first embodiment;
FIG. 31 is a perspective view showing a state before rotational engagement during the coupling of the handle unit and the sheath unit of the ultrasonic treatment apparatus in the first embodiment;
FIG. 32 is a plan view showing a state before the rotational engagement during the coupling of the handle unit and the sheath unit of the ultrasonic treatment apparatus in the first embodiment;
FIG. 33 is a perspective view showing a state after the rotational engagement during the coupling of the handle unit and the sheath unit of the ultrasonic treatment apparatus in the first embodiment;
FIG. 34 is a plan view showing a state after the rotational engagement during the coupling of the handle unit and the sheath unit of the ultrasonic treatment apparatus in the first embodiment;
FIG. 35A is a side view showing a state before a combination member is combined with a base member of a fixed handle of the handle unit of the ultrasonic treatment apparatus in the first embodiment;
FIG. 35B is a perspective view showing the switch unit of the ultrasonic treatment apparatus in the first embodiment;
FIG. 36 is a plan view showing the probe unit of the ultrasonic treatment apparatus in the first embodiment;
FIG. 37 is a sectional view along the 37-37 line in FIG. 36;
FIG. 38 is a plan view showing how the transducer unit of the ultrasonic treatment apparatus in the first embodiment is coupled to a cable;
FIG. 39 is a plan view showing the proximal end of a transducer unit cable of the ultrasonic treatment apparatus in the first embodiment;
FIG. 40 is a front view showing the distal end of the transducer unit of the ultrasonic treatment apparatus in the first embodiment;
FIG. 41 is a sectional view along the 41-41 line in FIG. 40;
FIG. 42 is a longitudinal sectional view showing the rear end of the transducer unit;
FIG. 43 is a sectional view along the 43-43 line in FIG. 41;
FIG. 44 is a sectional view along the 44-44 line in FIG. 42;
FIG. 45 is a sectional view along the 45-45 line in FIG. 42;
FIG. 46 is a perspective view showing how contact members and conducting plates of the transducer unit of the ultrasonic treatment apparatus in the first embodiment are disposed;
FIG. 47 is a perspective view showing a casing of the transducer unit of the ultrasonic treatment apparatus in the first embodiment;
FIG. 48 is a schematic configuration diagram showing electric paths of the transducer unit of the ultrasonic operating apparatus in the first embodiment;
FIG. 49 is a perspective view showing how the switch unit is attached to the fixed handle of the ultrasonic treatment apparatus in the first embodiment;
FIG. 50 is a perspective view showing, from a direction different from that in FIG. 49, how the switch unit is attached to the fixed handle of the ultrasonic treatment apparatus in the first embodiment;
FIG. 51 is a side view showing how the switch unit is attached to the fixed handle of the ultrasonic treatment apparatus in the first embodiment;
FIG. 52 is a side view showing how a switch of a handle of an operation portion of the ultrasonic treatment apparatus in the first embodiment is operated;
FIG. 53 is a side view of essential parts showing an ultrasonic treatment apparatus in a second embodiment of the present invention;
FIG. 54 is a side view of essential parts showing an ultrasonic treatment apparatus in a third embodiment of the present invention;
FIG. 55 is a side view of essential parts showing an ultrasonic treatment apparatus in a fourth embodiment of the present invention;
FIG. 56 is a schematic configuration diagram showing how a power supply main unit and hand piece of an ultrasonic treatment apparatus in a fifth embodiment of the present invention are connected together;
FIG. 57 is a schematic configuration diagram showing internal electric wiring lines of a connector portion provided in a cable of the hand piece of the ultrasonic treatment apparatus in the fifth embodiment;
FIG. 58 is a side view showing the configuration of essential parts of an ultrasonic treatment apparatus in a sixth embodiment of the present invention; and
FIG. 59 is a side view showing the configuration of essential parts of an ultrasonic treatment apparatus in a seventh embodiment of the present invention.

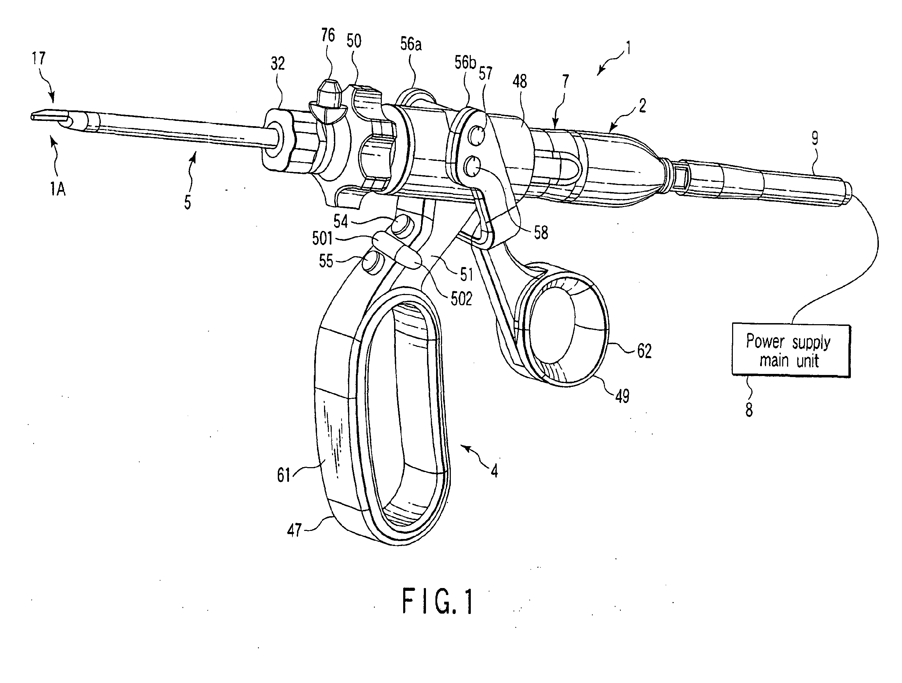

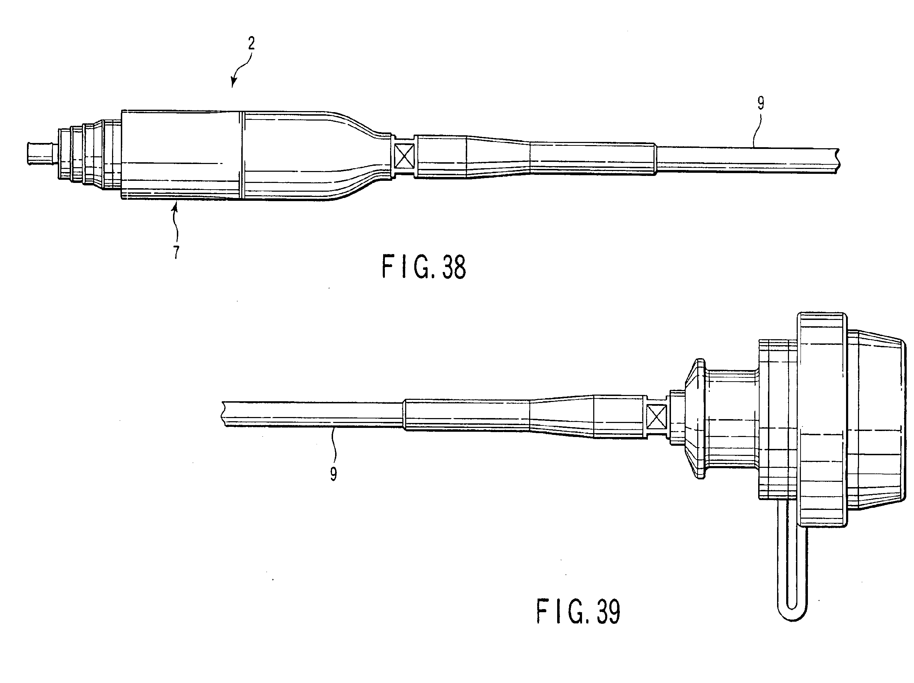

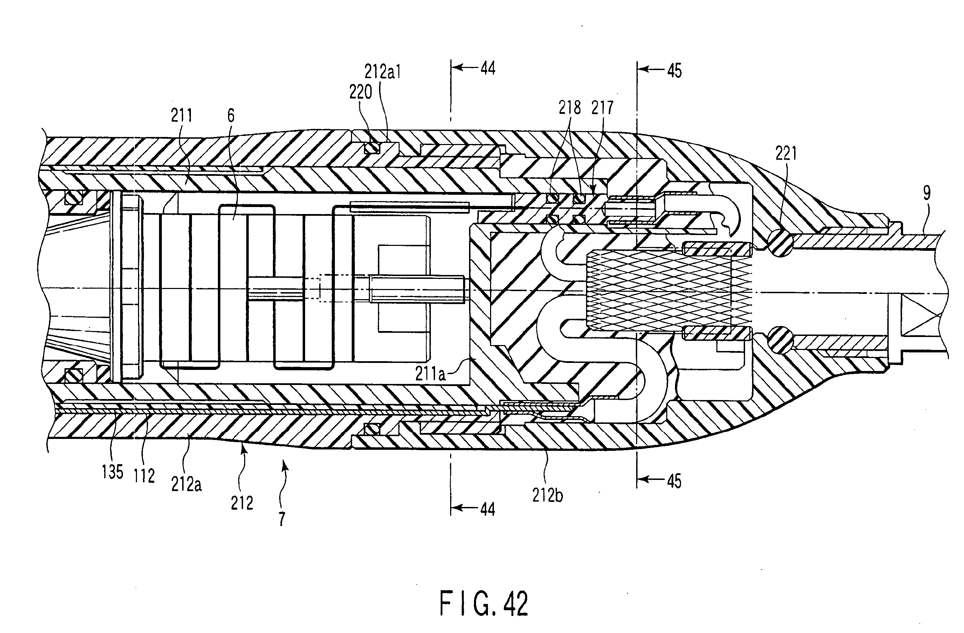

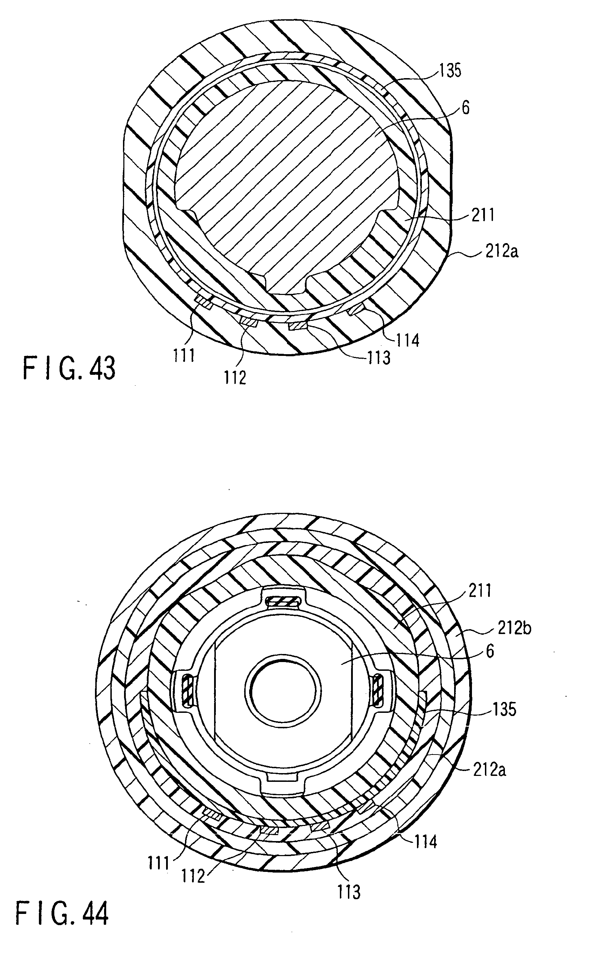

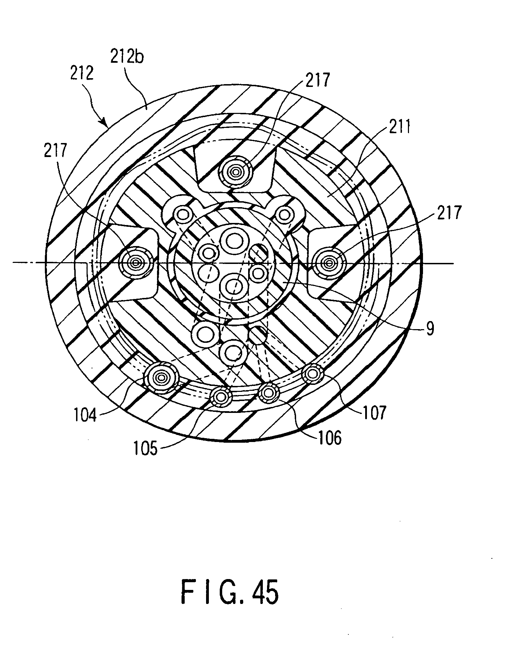

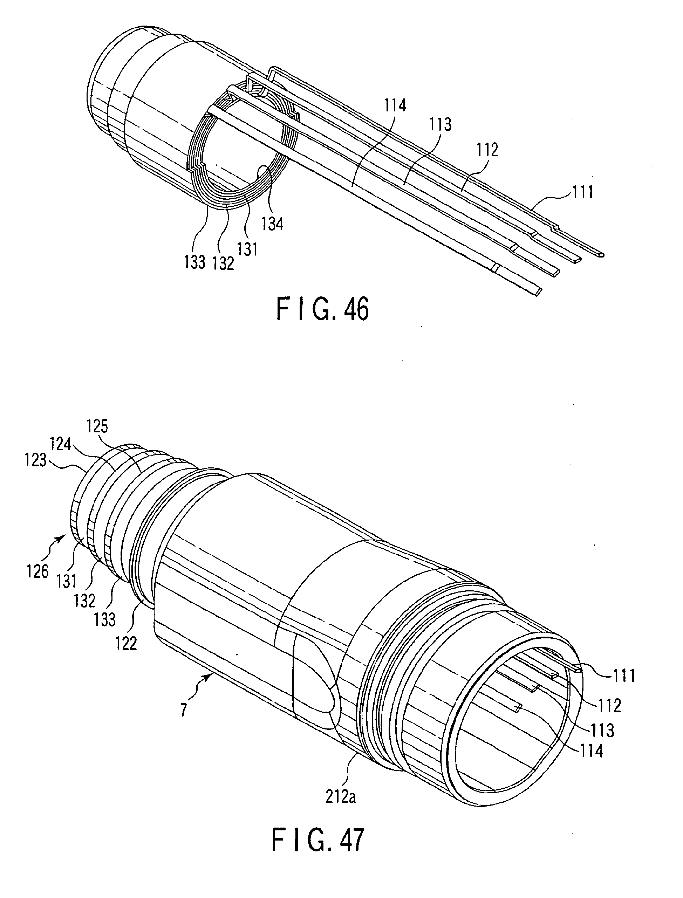

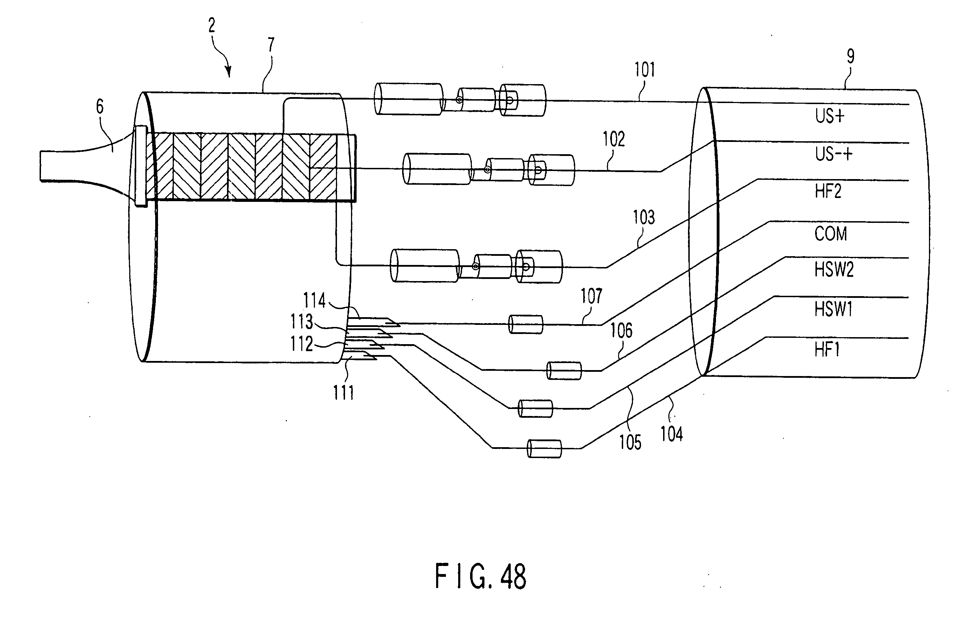

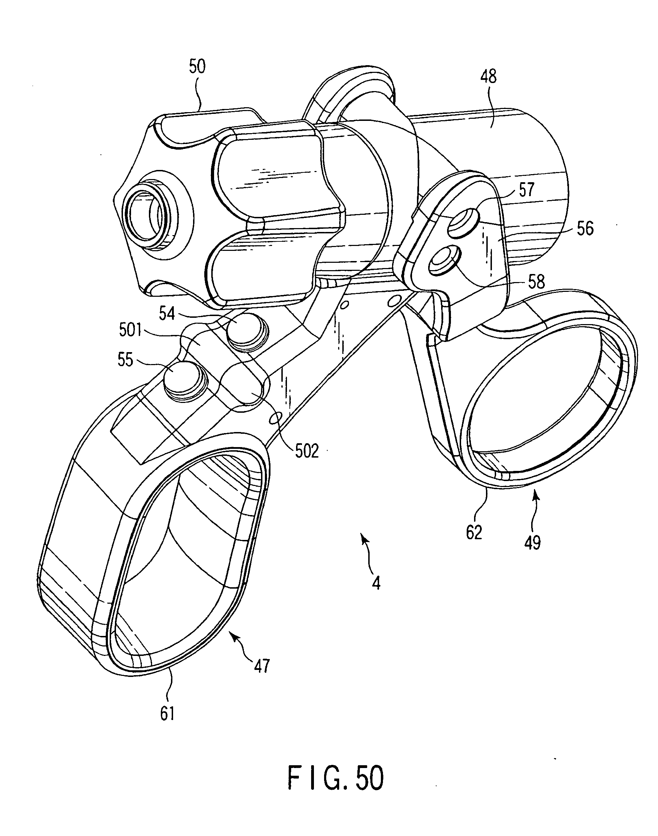

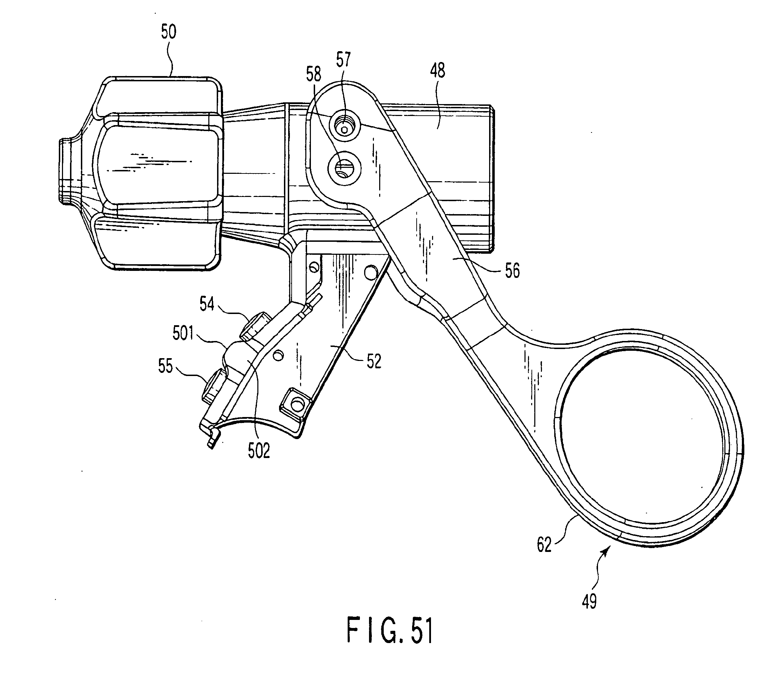

Hereinafter, a first embodiment of the present invention will be described with reference to FIG. 1 to FIG. 52. FIG. 1 shows the schematic configuration of a whole hand piece 1 of an ultrasonic treatment apparatus which is a surgical apparatus in the present embodiment. The ultrasonic treatment apparatus in the present embodiment is an ultrasonic coagulation/incision treatment apparatus capable of administering a treatment such as incision, removal or coagulation of a living tissue by use of ultrasonic waves and also capable of administering a treatment with a high frequency.

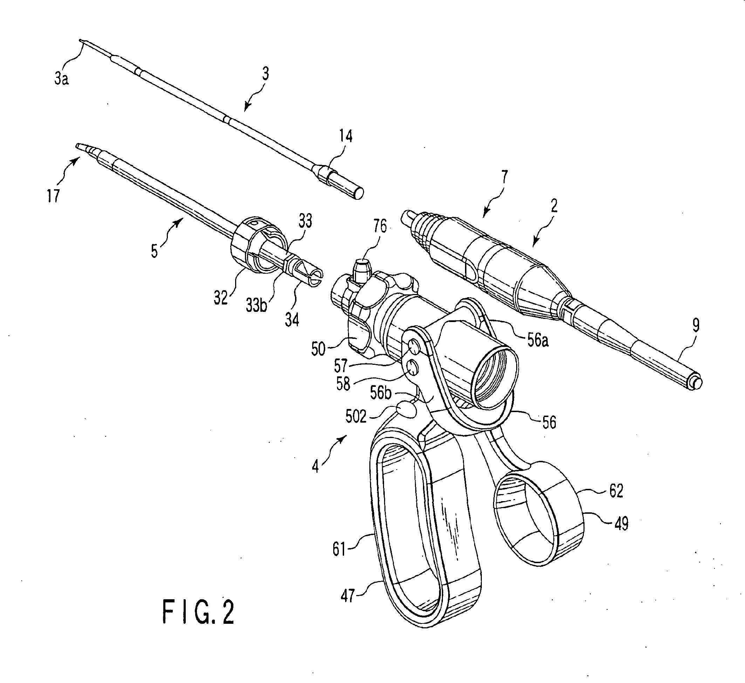

As shown in FIG. 2, the hand piece 1 has four units: a transducer unit 2, a probe unit (probe portion) 3, a handle unit (operation portion) 4, and a sheath unit (sheath portion) 5. These four units are removably coupled to each other.

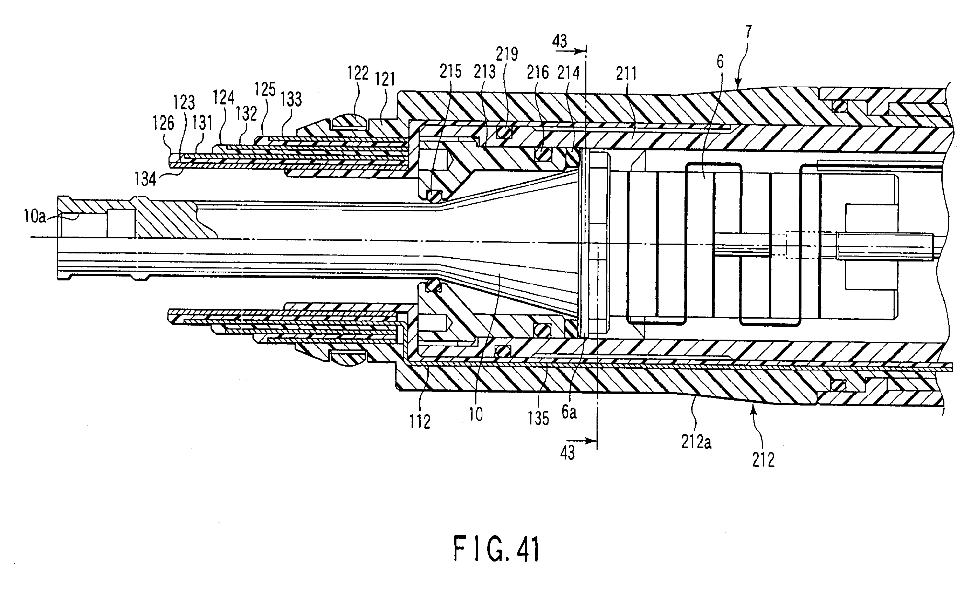

In the transducer unit 2, there is incorporated a transducer 6 (see FIG. 41) described later for generating ultrasonic vibrations by a piezoelectric element which converts an electric current into the ultrasonic vibrations. The outside of the piezoelectric element is covered with a cylindrical transducer cover 7. Further, at the rear end of the transducer unit 2, a cable 9 extends to supply from a power supply main unit 8 an electric current for generating the ultrasonic vibrations.

The proximal end of a horn 10 for amplifying/expanding the ultrasonic vibrations is coupled to the front end of the ultrasonic transducer 6 within the transducer cover 7. A screw hole 10a for attaching a probe is formed at the distal end of the horn 10.

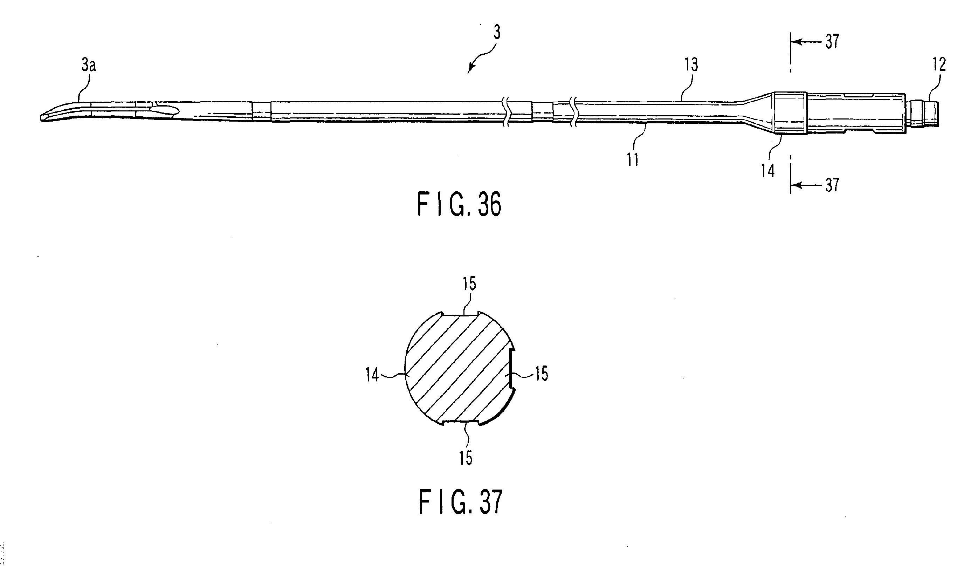

FIG. 36 shows an overall external appearance of the probe unit 3. This probe unit 3 is designed so that its entire length may be the integral multiple of the half-wave length of the ultrasonic vibrations. The probe unit 3 has a rod-like vibration transmitting member 11 made of a metal which has a distal end and a proximal end and which has a long axis. A screw portion 12 for screwing into the screw hole 10a of the horn 10 is provided at the proximal end of the vibration transmitting member 11. Further, this screw portion 12 is threadably attached to the screw hole 10a of the horn 10 in the transducer unit 2. This sets the probe unit 3 and the transducer unit 2 together. At this point, a first high-frequency electric path 13 for transmitting a high-frequency current is formed in a combination of the ultrasonic transducer 6 and the probe unit 3.

A probe distal end 3a is provided at the distal end of the vibration transmitting member 11. The probe distal end 3a is formed to have a substantially J-shaped curve. The axial sectional area of the probe unit 3 is reduced at several vibration nodes partway in the axial direction so that amplitude necessary for a treatment can be obtained at the probe distal end 3a. Rubber rings formed of an elastic member with a ring shape are attached at several positions of the vibration nodes partway in the axial direction of the probe unit 3. Thus, these rubber rings prevent interference between the probe unit 3 and the sheath unit 5.

A flange portion 14 is provided at the position of the vibration node closest to the side of the proximal end in the axial direction of the probe unit 3. As shown in FIG. 37, keyway-shaped engaging concave portions 15 are formed on the outer peripheral surface of this flange portion 14 at three places in a circumferential direction.

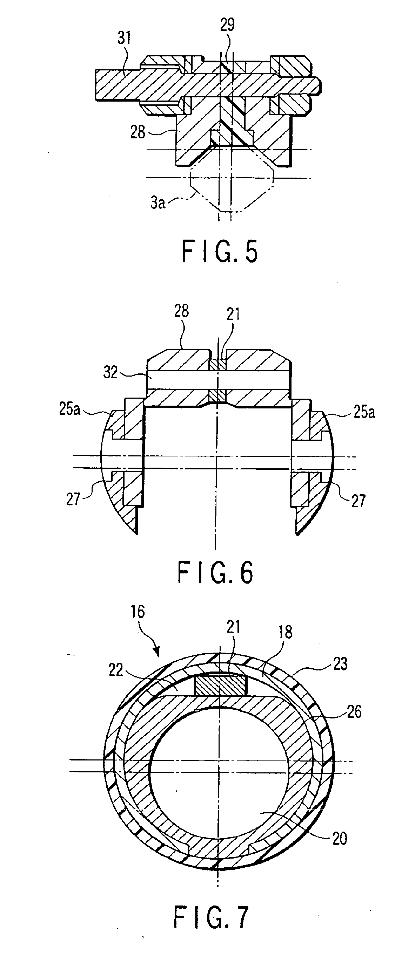

The sheath unit 5 has a sheath main unit 16 formed by a cylindrical member, and a jaw 17 disposed at the distal end of the sheath main unit 16. The sheath main unit 16 has a metal outer cylinder 18 whose sectional shape is circular as shown in FIG. 7, and a metal inner cylinder 19 whose sectional shape is non-circular, for example, D-shaped. A channel 22 for passing a drive shaft 21 of the jaw 17 is formed between the outer cylinder 18 and the inner cylinder 19.

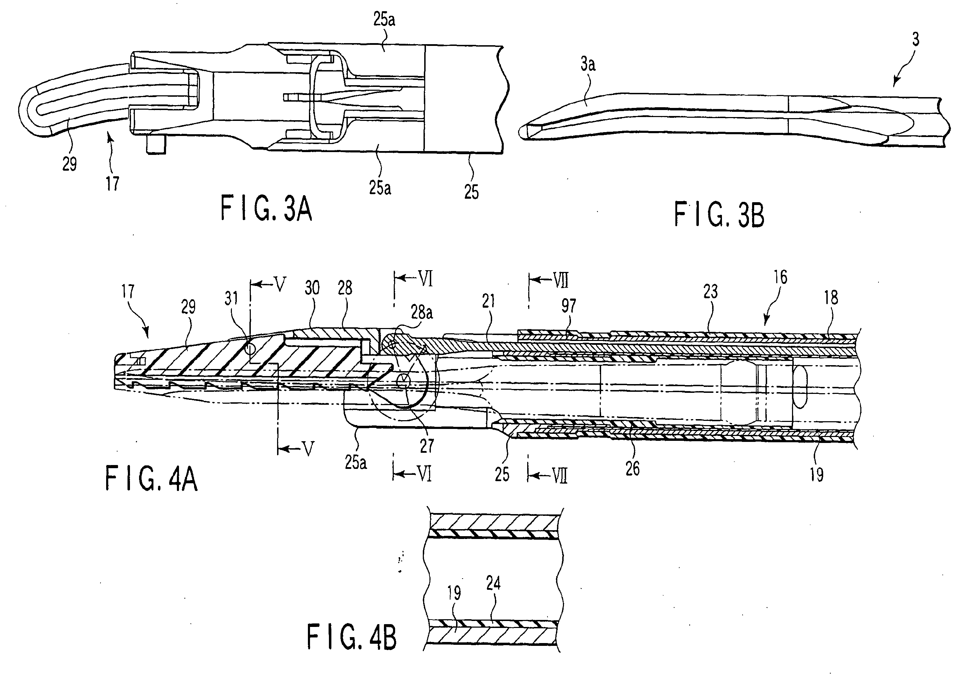

As shown in FIG. 4A, the outer peripheral surface of the outer cylinder 18 is covered with an insulating tube 23. As shown in FIG. 4B, an insulating coating 24 is formed by an insulating material on the inner peripheral surface of the inner cylinder 19. In addition, an insulating tube may be provided on the inner peripheral surface of the inner cylinder 19. Thus, the inner cylinder 19 is electrically insulated from the probe unit 3 by the insulating coating 24.

The proximal end of a substantially cylindrical distal end cover 25 is fixed to the distal end of the outer cylinder 18. On the side of the inner peripheral surface of the proximal end of the distal end cover 25, there is attached a pipe-shaped holding member 26 for holding the probe unit 3 to prevent this probe unit 3 from contacting the distal end cover 25. A channel 20 having a circular section for passing the probe unit 3 is formed inside the holding member 26.

As shown in FIG. 3A, a pair of right and left jaw support portions 25a is provided at the distal end of the distal end cover 25 to extend forward from the outer cylinder 18. A metal jaw main unit 28 of the jaw 17 is swingably attached to these jaw support portions 25a via two supporting point pins 27, as shown in FIG. 6. This jaw 17 is formed to have a substantially J-shaped curve corresponding to the probe distal end 3a of the probe unit 3, as shown in FIG. 3A.

Thus, the jaw 17 is opposite to the probe distal end 3a of the probe unit 3 and swingably supported on the two supporting point pins 27 (see FIG. 6). The jaw 17 is operated to swing between an open position at which the jaw 17 swings in a direction to move away from the probe distal end 3a of the probe unit 3 and a closing position at which the jaw 17 swings in a direction to approach the side of the probe distal end 3a of the probe unit 3. If the jaw 17 is operated to swing to the closing position, the living tissue is gripped between the jaw 17 and the probe distal end 3a of the probe unit 3.

A treatment portion 1A of the hand piece 1 is formed by the jaw 17 and the probe distal end 3a of the probe unit 3. The treatment portion 1A has a plurality of, in the present embodiment, two selectable surgical functions (a first surgical function and a second surgical function). For example, the first surgical function is set to a function for simultaneously outputting an ultrasonic treatment output and a high-frequency treatment output. The second surgical function is set to a function for independently outputting the high-frequency treatment output alone.

In addition, the first surgical function and the second surgical function of the treatment portion 1A are not limited to the configurations mentioned above. For example, the first surgical function may be set to a function for outputting the ultrasonic treatment output in a maximum output state, and the second surgical function may be set to a function for outputting the ultrasonic treatment output in a preset arbitrary set output state lower than the maximum output state.

The jaw main unit 28 has a grip member 29 made of a resin such as PTFE, and a metal grip member attachment member 30 for holding the grip member 29. The grip member 29 is attached to the grip member attachment member 30 so that this grip member 29 can swing over a given angle by a pin 31 (see FIG. 5). Further, the distal end of the drive shaft 21 is coupled to the rear end of the jaw main unit 28 via a pin 28a, as shown in FIG. 4A. This drive shaft 21 passes inside the distal end cover 25, and then passes between the outer cylinder 18 and the inner cylinder 19 of the sheath main unit 16 as shown in FIG. 7, thus extending out to the side of the proximal end of the sheath main unit 16.

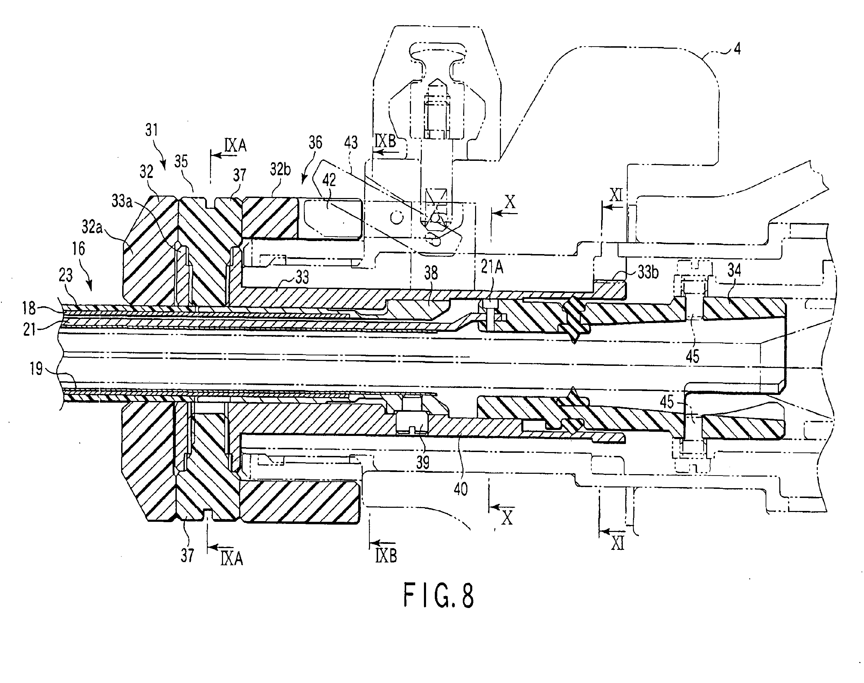

FIG. 8 shows the proximal end of the sheath main unit 16. An attachment/detachment mechanism section 31 for attachment to/detachment from the handle unit 4 is provided at the proximal end of the sheath main unit 16. The attachment/detachment mechanism section 31 has a cylindrical large-diameter pinch member 32 formed of a resin material, a guide cylindrical member 33 formed by a metal cylindrical member, and a cylindrical connecting pipe member 34 formed of a resin material.

The pinch member 32 has a first ring-shaped fixing portion 32a disposed at the front end, and a second cylindrical fixing portion 32b disposed at the rear end. The inner peripheral surface of the first fixing portion 32a is fixed to the outer peripheral surface of the proximal end of the sheath main unit 16. The second fixing portion 32b of the pinch member 32 has a fixing portion 35 of the guide cylindrical member 33 disposed on the front end side, and an attachment/detachment portion 36 disposed on the rear end side for attachment to/detachment from the handle unit 4.

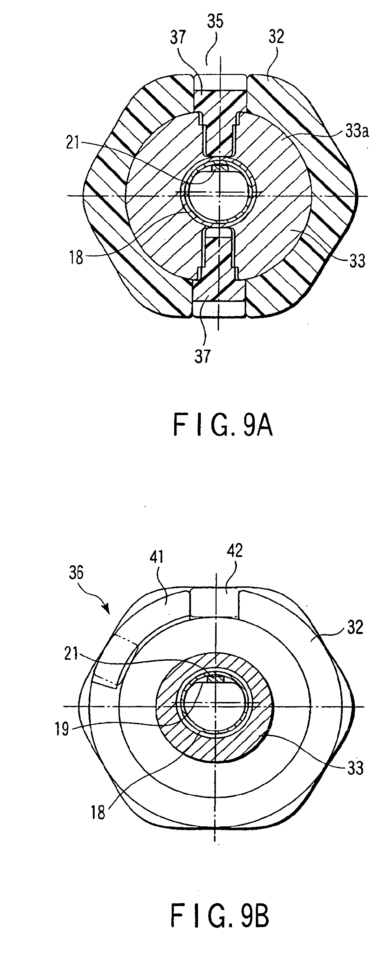

The guide cylindrical member 33 has a large-diameter front end flange portion 33a disposed at the front end, and an outer peripheral flange portion 33b disposed on the rear end side. As shown in FIG. 9A, the front end flange portion 33a of the guide cylindrical member 33 is fixed to the pinch member 32 by two fixing screws 37 made of a resin while being inserted in the pinch member 32.

A metal joining pipe 38 is disposed inside the guide cylindrical member 33. The inner peripheral surface at the front end of this joining pipe 38 is fixed to the outer cylinder 18 of the sheath main unit 16 by laser welding. Further, the joining pipe 38 is fixed to the guide cylindrical member 33 by a metal fixing screw 39. This permits electric conduction between the guide cylindrical member 33,' the fixing screw 39, the joining pipe 38, the outer cylinder 18, the distal end cover 25, the supporting point pins 27 and the jaw main unit 28, thereby forming a sheath unit side electric path 40 for transmitting a high-frequency current.

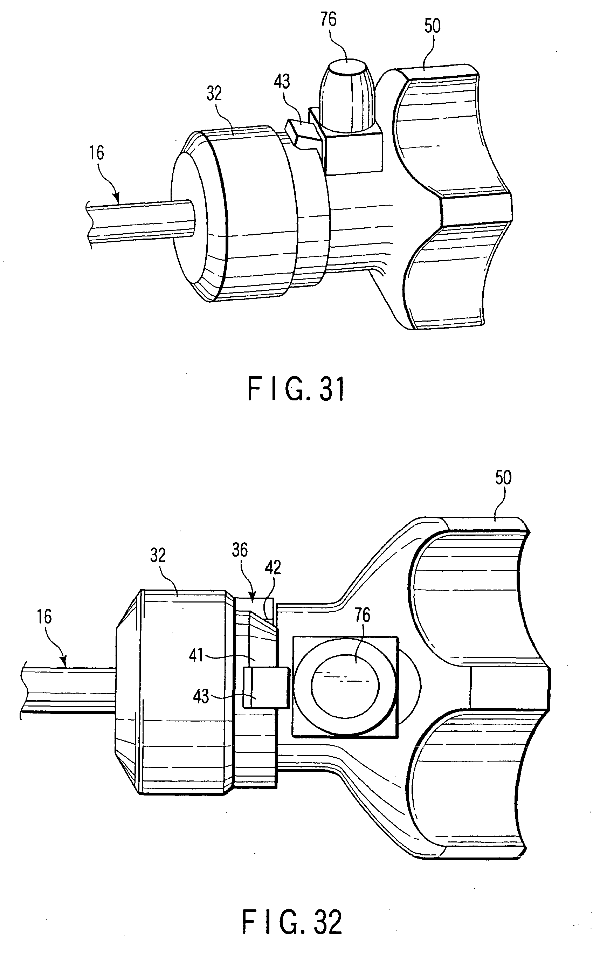

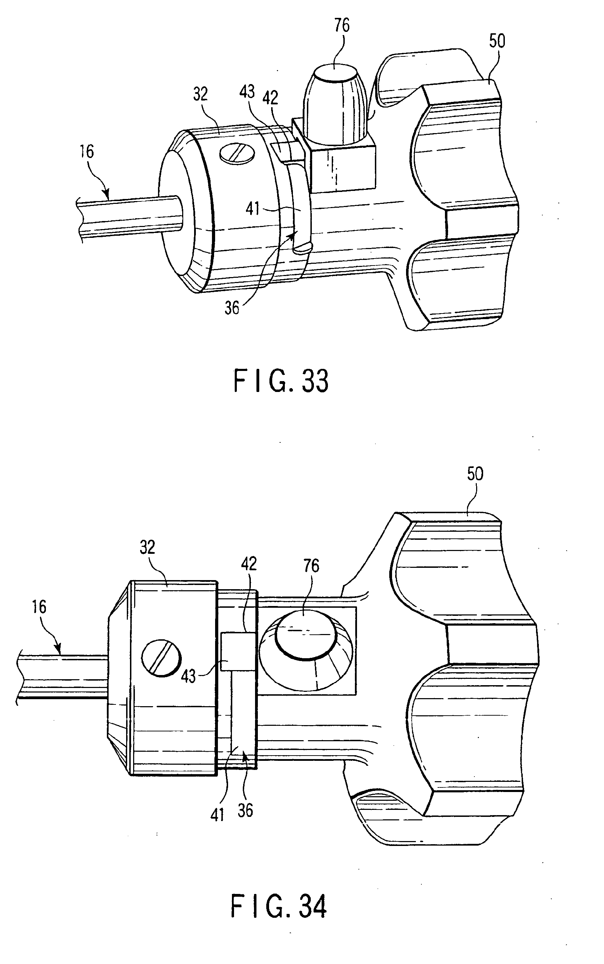

The attachment/detachment portion 36 of the pinch member 32 has a guide groove 41 in the form of an inclined surface provided to extend along a circumferential direction as shown in FIG. 9B, and an engaging concave portion 42 formed at one end of this guide groove 41. The guide groove 41 has a tapered inclined surface whose outside diameter becomes smaller as it approaches the side of the rear end of the pinch member 32. The engaging concave portion 42 is formed by a recessed portion whose diameter is smaller than that of the inclined surface of the guide groove 41. An engaging lever 43 described later on the side of the handle unit 4 removably engages with the engaging concave portion 42. FIGS. 33 and 34 show how the engaging lever 43 engages with the engaging concave portion 42, and FIGS. 31 and 32 show a disengaged state in which the engaging lever 43 is pulled out of the engaging concave portion 42.

The connecting pipe member 34 is inserted into the guide cylindrical member 33 slidably in a direction of the axis line of the sheath main unit 16. The proximal end of the drive shaft 21 is fixed to the distal end of this connecting pipe member 34 via a pin 21A (see

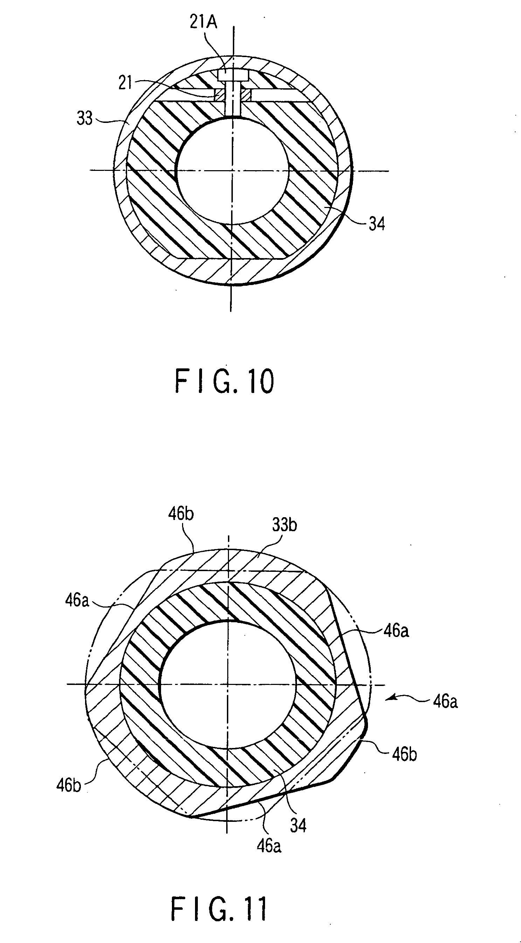

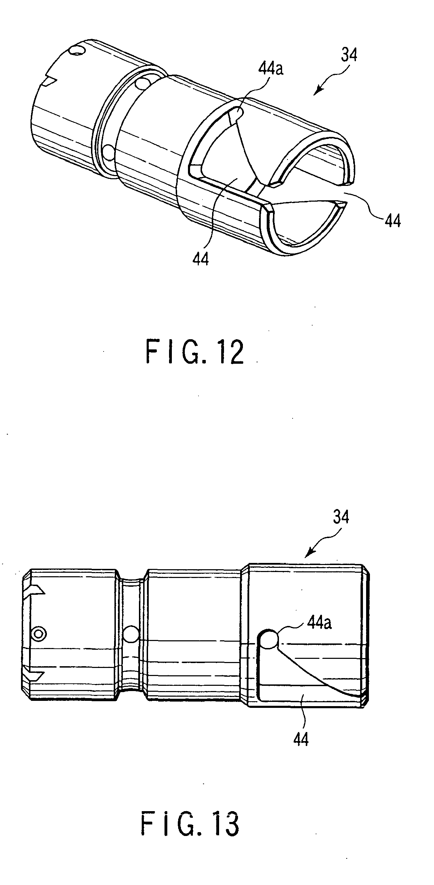

FIG. 10). Two guide grooves 44 shown in FIGS. 12 and 13 are provided at the proximal end of the connecting pipe member 34. Engaging pins 45 described later on the side of the handle unit 4 removably engage with the guide grooves 44. At the terminal end of the guide groove 44, there is formed an engaging groove 44a which regulates the movement of the engaging pin 45 in the direction of the axis line of the sheath main unit 16.

The outer peripheral flange portion 33b has a non-circular engaging portion 46. In the engaging portion 46, there are formed three plane portions 46a formed by cutting off a plurality of places, three places in the present embodiment, in the circular outer peripheral surface of the outer peripheral flange portion 33b. Corner portions 46b whose diameters are larger than those of the plane portions 46a are formed at junctions between the three plane portions 46a. Thus, the engaging portion 46 whose sectional shape is substantially close to a triangular shape is formed in the outer peripheral flange portion 33b. In addition, this non-circular engaging portion 46 does not necessarily have to have the substantially triangular shape, and various shapes including polygonal shapes such as quadrangular and pentangular shapes can be conceived as long as they are non-circular shapes.

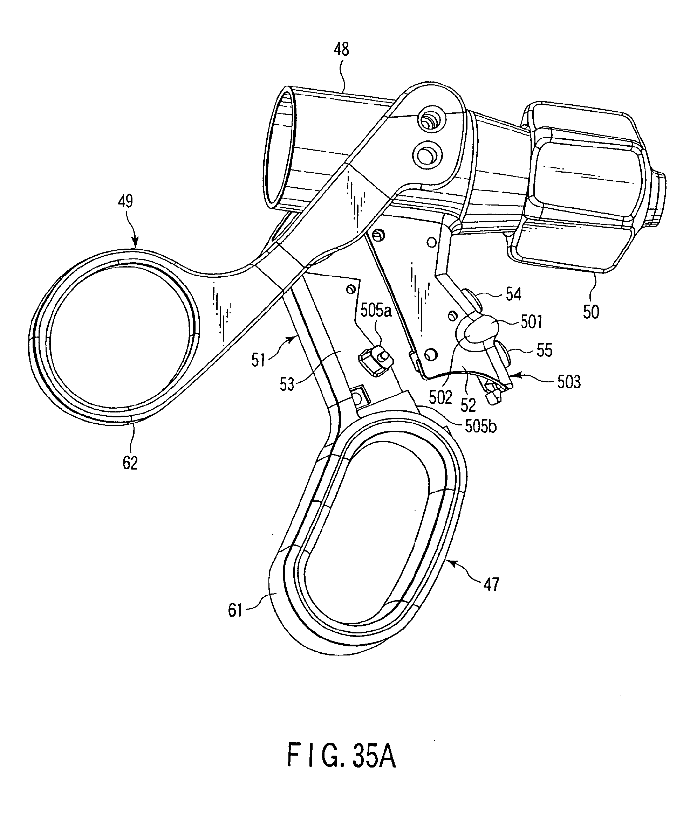

The handle unit 4 mainly has a fixed handle (fixed handle element) 47, a holding cylinder 48, a movable handle (movable handle element) 49, a swing operation knob 50, and a handle unit side electric path 95 for transmitting a high-frequency current. The holding cylinder 48 is disposed on the top of the fixed handle 47. A switch holding portion 51 is provided between the fixed handle 47 and the holding cylinder 48. As shown in FIG. 35A, the switch holding portion 51 has a switch attachment portion 52 fixed to the lower end of the holding cylinder 48, and a cover member 53 fixed to the upper end of the fixed handle 47.

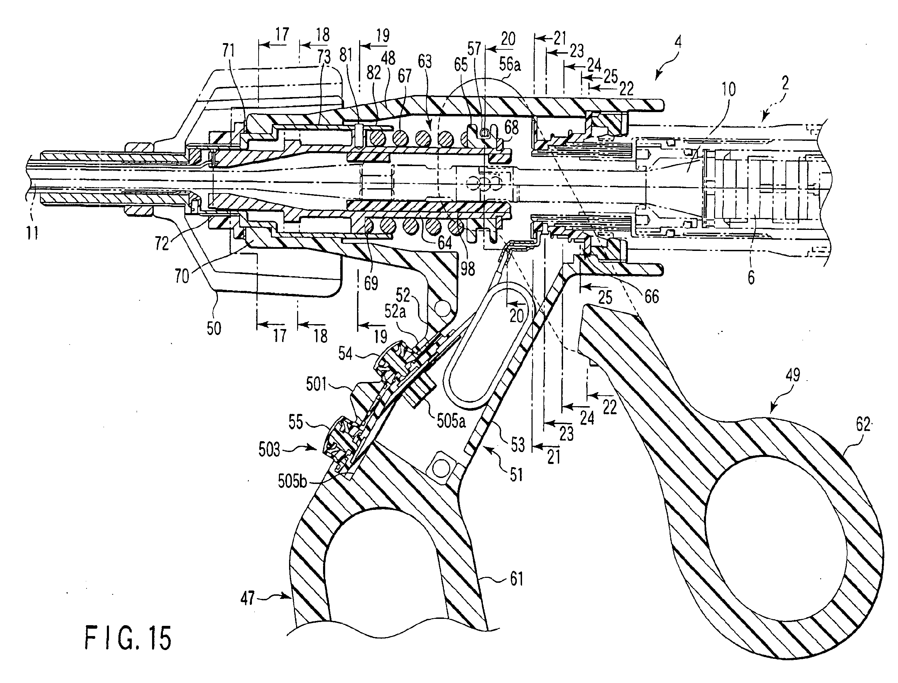

As shown in FIG. 15, the switch attachment portion 52 has, on its front side, a switch attachment surface 52a for attaching a plurality of switches, in the present embodiment, two switches (a first switch 54 and a second switch 55). The first switch 54 and the second switch 55 are switches for selecting the surgical functions of the treatment portion 1A of the hand piece 1.

In the switch attachment portion 52, the first switch 54 and the second switch 55 are vertically arranged. Further, on the switch attachment surface 52a, a bulging portion 501 is disposed between the first switch 54 and the second switch 55. The bulging portion 501 divides the switches 54 and 55, and doubles as a finger receiving portion.

The first switch 54 is disposed on the upper side of the switch attachment surface 52a, and set to a switch for selecting the frequently used first surgical function of the plurality of surgical functions. The second switch 55 is disposed on the lower side of the switch attachment surface 52a, and set to a switch for selecting another second surgical function of the plurality of surgical functions.

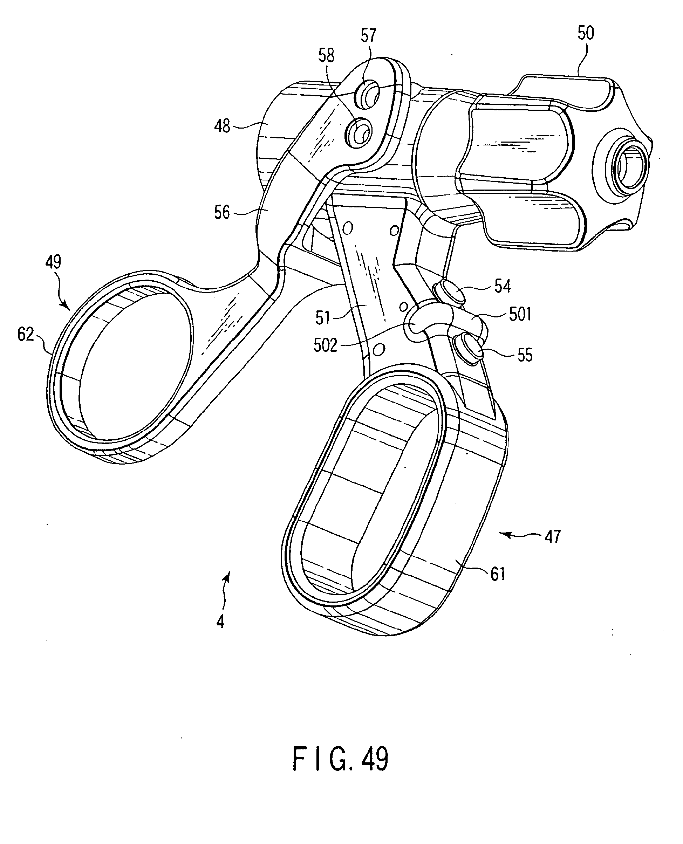

The bulging portion 501 is set so that the height of projection of this bulging portion from the switch attachment surface 52a is larger than the height of projection of the first switch 54 and the second switch 55 from the attachment surface 52a. The bulging portion 501 has an extension 502 (see FIGS. 49 to 51) which continuously extends from the switch attachment surface 52a of the fixed handle 47 over both lateral sides thereof.

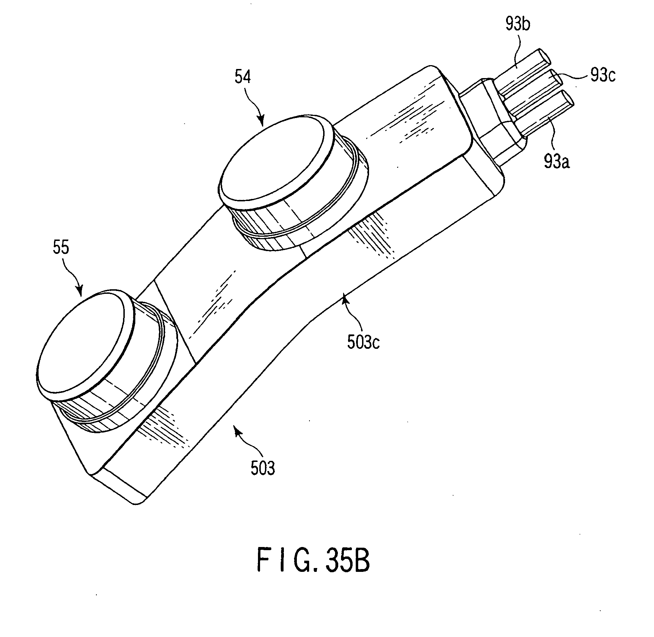

The switch attachment portion 52 has one switch unit 503, and a concave unit receiver 504 to which the switch unit 503 is attached. As shown in FIG. 35B, the switch unit 503 includes two switches (the first switch 54 and the second switch 55) that are integrated into one unit.

The switch unit 503 has a push button 54a for the first switch 54, a push button 55a for the second switch 55, a flexible wiring line circuit board 503a for the two switches (the first switch 54 and the second switch 55), and a flexible base member 503c in which the wiring line circuit board 503a is embedded in two insulating rubber plates (elastic members) 503b.

Connected to the wiring line circuit board 503a are a first surgical function wiring line 93a whose one end is connected to the first switch 54, a second surgical function wiring line 93b whose one end is connected to the second switch 55, and a ground wiring line 93c whose one end is connected to a common terminal for ground. These three wiring lines 93a to 93c are incorporated in the switch holding portion 51 in a rolled state.

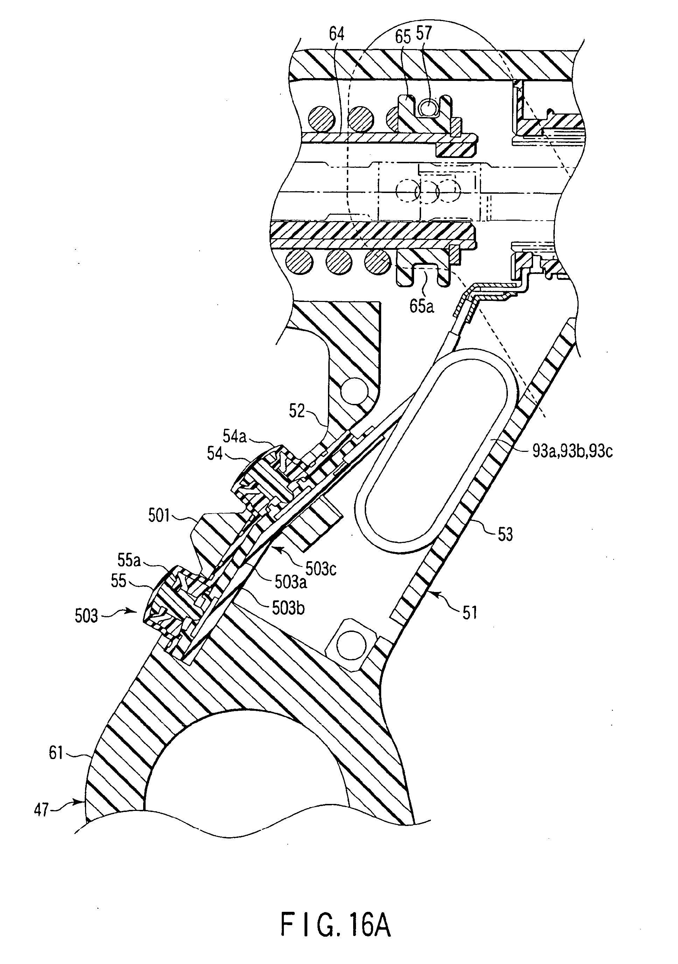

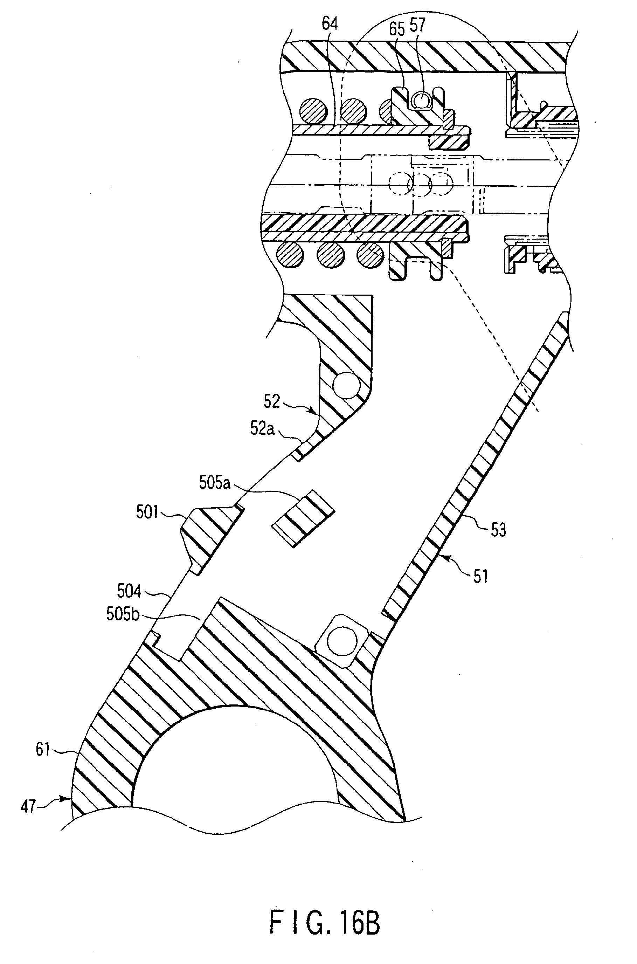

As shown in FIG. 16B, the unit receiver 504 has two bosses 505a and 505b for receiving force to push the push buttons 54a and 55a for the two switches. One boss 505a is disposed in a part corresponding to the push button 54a for the first switch 54. The other boss 505b is disposed in a part corresponding to the push button 55a for the second switch 55. Thus, the force to push the push button 54a for the first switch 54 is received by the boss 505a, and the force to push the push button 55a for the second switch 55 is received by the boss 505b.

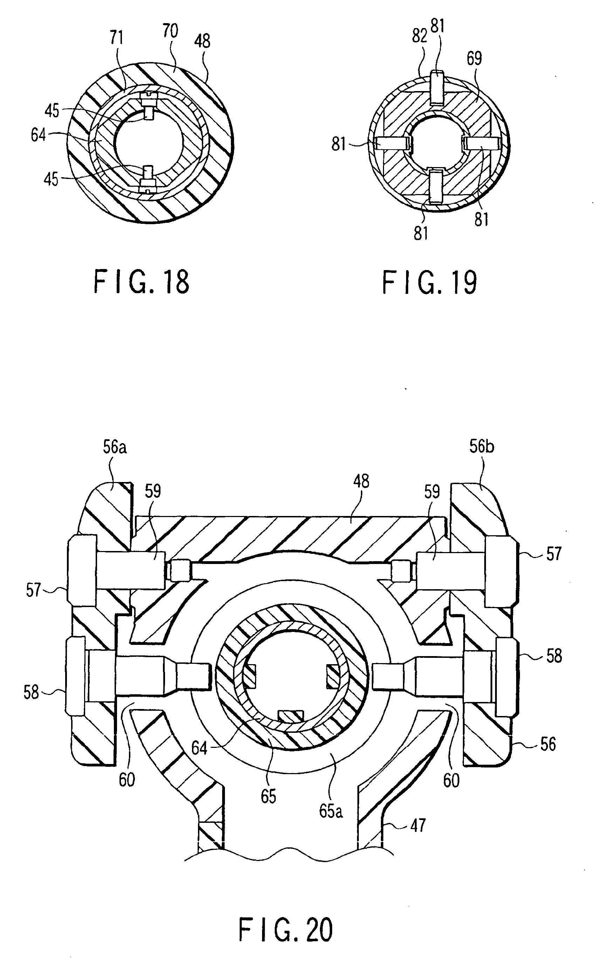

A movable handle 49 has a substantially U-shaped arm portion 56 on its top. The U-shaped arm portion 56 has two arms 56a and 56b, as shown in FIG. 20. The movable handle 49 is set to the holding cylinder 48 so that the holding cylinder 48 is inserted between the two arms 56a and 56b.

Each of the arms 56a and 56b has a supporting point pin 57 and an action pin 58. Pin receiving holes 59 and windows 60 are formed on both sides of the holding cylinder 48. The supporting point pin 57 of each of the arms 56a and 56b is inserted in the pin receiving hole 59 of the holding cylinder 48. Thus, the upper end of the movable handle 49 is swingably supported on the holding cylinder 48 via the supporting point pins 57.

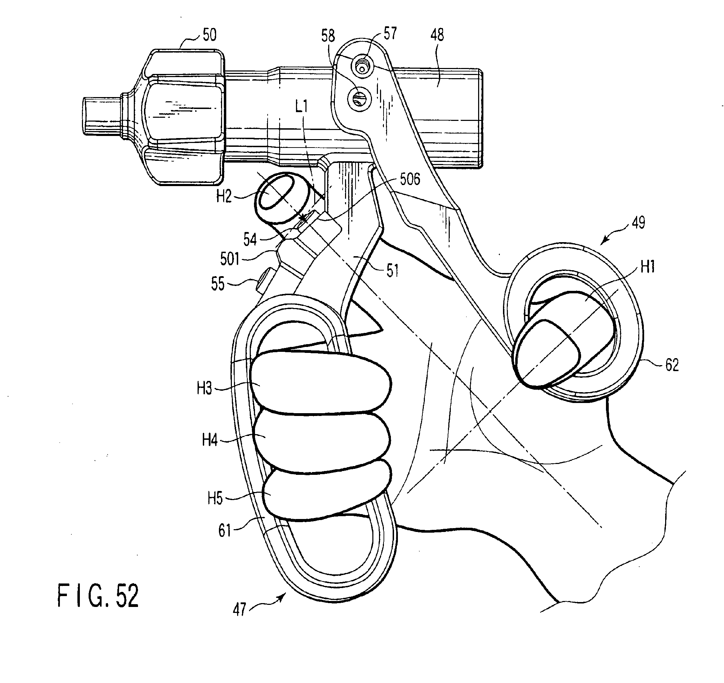

As shown in FIG. 52, the movable handle 49 has a thumb insertion ring portion 62 into which a thumb H1 of a user is inserted. The fixed handle 47 has a multiple finger insertion ring portion 61 into which a plurality of fingers H3, H4 and H5 except for the thumb H1 and an index finger H2 are inserted. Thus, the handles are gripped by the fingers put on these portions, such that the movable handle 49 swings via the supporting point pins 57, and the movable handle 49 opens/closes with respect to the fixed handle 47.

The switch attachment surface 52a has a curving surface 506 curving along a flow line L1 on which the index finger H2 moves in a condition where the thumb H1 is inserted into the thumb insertion ring portion 62 and the plurality of fingers H3, H4 and H5 except for the thumb H1 and index finger H2 are inserted into the multiple finger insertion ring portion 61, as shown in FIG. 52. The switch unit 503 is attached to the unit receiver 504 so that the base member 503c curves along the curving surface 506.

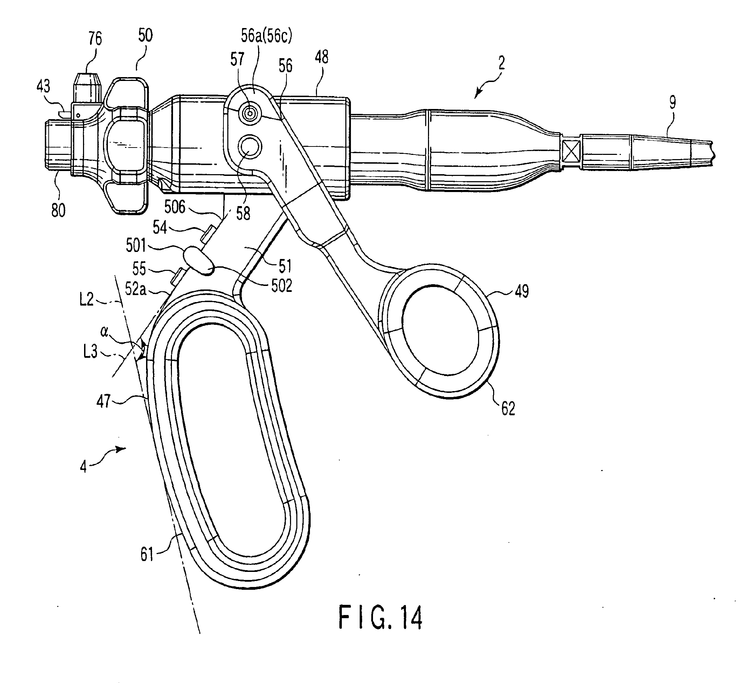

As shown in FIG. 14, the handle unit 4 is set so that an angle α between a tangent line L2 of a front surface of the multiple finger insertion ring portion 61 of the fixed handle 47 and a tangent line L3 of a front surface of the switch attachment surface 52a is larger than 90°.

Each of the action pins 58 of the movable handle 49 extends into the holding cylinder 48 through a window 60 of the holding cylinder 48. An operation force transmitting mechanism 63 for transmitting the operation force of the movable handle 49 to the drive shaft 21 of the jaw 17 is provided inside the holding cylinder 48.

As shown in FIG. 15, the operation force transmitting mechanism 63 has a cylindrical spring bearing member 64 mainly made of a metal, and a slider member 65 made of a resin. The spring bearing member 64 is disposed coaxially with the central line of the holding cylinder 48, and provided to extend in the same direction as the insertion direction of the probe unit 3.

On the outer peripheral surface of the spring bearing member 64, there are provided a coil spring 67, the slider member 65, a stopper 68 and a spring bearing 69. The front end of the coil spring 67 is fixed to the spring bearing 69. The stopper 68 regulates the moving position of the rear end side of the slider member 65. The coil spring 67 is installed between the spring bearing 69 and the slider member 65 with a given amount of force of equipment.

A ring-shaped engaging groove 65a is formed on the outer peripheral surface of the slider member 65 along its circumferential direction. The action pins 58 of the movable handle 49 engage with the engaging groove 65a so that they are inserted in this engaging groove 65a, as shown in FIG. 20. Thus, when the movable handle 49 is gripped to close the movable handle 49 with respect to the fixed handle 47, the movable handle 49 swings so that the action pins 58 swing around the supporting point pins 57. The slider member 65 interlocked with the swing operation of the supporting point pins 57 moves forward along the axial direction. At this point, the spring bearing member 64 coupled to the slider member 65 via the coil spring 67 also moves back and forth together with the slider member 65. Thus, the operation force of the movable handle 49 is transmitted to the connecting pipe member 34 via the pair of engaging pins 45, and the drive shaft 21 of the jaw 17 moves forward. Therefore, the jaw main unit 28 of the jaw 17 swings via the supporting point pins 27.

Furthermore, when the living tissue is gripped between the grip member 29 of the jaw 17 and the probe distal end 3a of the probe unit 3 in accordance with the above operation, the grip member 29 swings at a given angle on the pin 31A to follow the bending of the probe distal end 3a so that force is equally applied to the overall length of the grip member 29. When the ultrasonic waves are output in this state, it is possible to coagulate or incise the living tissue such as a blood vessel.

A ring-shaped bearing 70 is formed at the front end of the holding cylinder 48. A cylindrical rotation transmitting member 71 made of a metal is coupled to the bearing 70 swingably in a direction around the axis. In the rotation transmitting member 71, there are formed a protrusion 72 protruding ahead of the bearing 70, and a large-diameter portion 73 provided to extend from the bearing 70 onto the internal side of the holding cylinder 48.

The swing operation knob 50 is fixed to the protrusion 72 in an externally fitted state. The engaging lever 43 is provided at the front end of this swing operation knob 50. The intermediate portion of the engaging lever 43 is swingably coupled to the protrusion 72 via a pin 74. The proximal end of the engaging lever 43 extends into the inside of a lever receiving concave portion 75 formed in the front surface of the swing operation knob 50.

An operation button 76 for operating the engaging lever 43 in a disengaging direction is provided on the outer peripheral surface at the front end of the swing operation knob 50. A downward actuating pin 77 is provided to protrude in the operation button 76. The actuating pin 77 extends onto the internal side of the lever receiving concave portion 75 via a wall hole of the swing operation knob 50. The proximal end of the engaging lever 43 is swingably coupled to the lower end of the actuating pin 77 via a pin 78.

A drop preventing ring 80 for the swing operation knob 50 is provided at the distal end of the protrusion 72. A male screw 79 is formed at the distal end of the protrusion 72. A female screw 80a to which the male screw 79 is threadably attached is formed on the inner peripheral surface of the drop preventing ring 80. Thus, the female screw 80a of the drop preventing ring 80 is screwed to the male screw 79 of the protrusion 72, such that the swing operation knob 50 is fixed to the rotation transmitting member 71.

As shown in FIG. 19, four positioning pins 81 made of a metal are provided to diametrically outwardly protrude in the spring bearing 69 of the spring bearing member 64. A long-hole-shaped engaging hole 82 into which one pin 81 of the spring bearing member 64 is inserted is formed in the large-diameter portion 73 of the rotation transmitting member 71. The engaging hole 82 is provided to extend in the same direction as the insertion direction of the probe unit 3. Thus, the pin 81 is moved along the engaging hole 82 during the operation of the movable handle 49, thereby preventing the back-and-forth movement of the spring bearing member 64 from being transmitted to the rotation transmitting member 71.

On the contrary, the rotational operation of the rotation transmitting member 71 rotating together with the swing operation knob 50 is transmitted to the side of the spring bearing member 64 via the pin 81 during the rotational operation of the swing operation knob 50. Thus, during the rotational operation of the swing operation knob 50, a set unit including the rotation transmitting member 71, the pin 81, the spring bearing member 64, the slider member 65 and the coil spring 67 inside the holding cylinder 48 is driven to integrally rotate in a direction around the axis together with the swing operation knob 50.

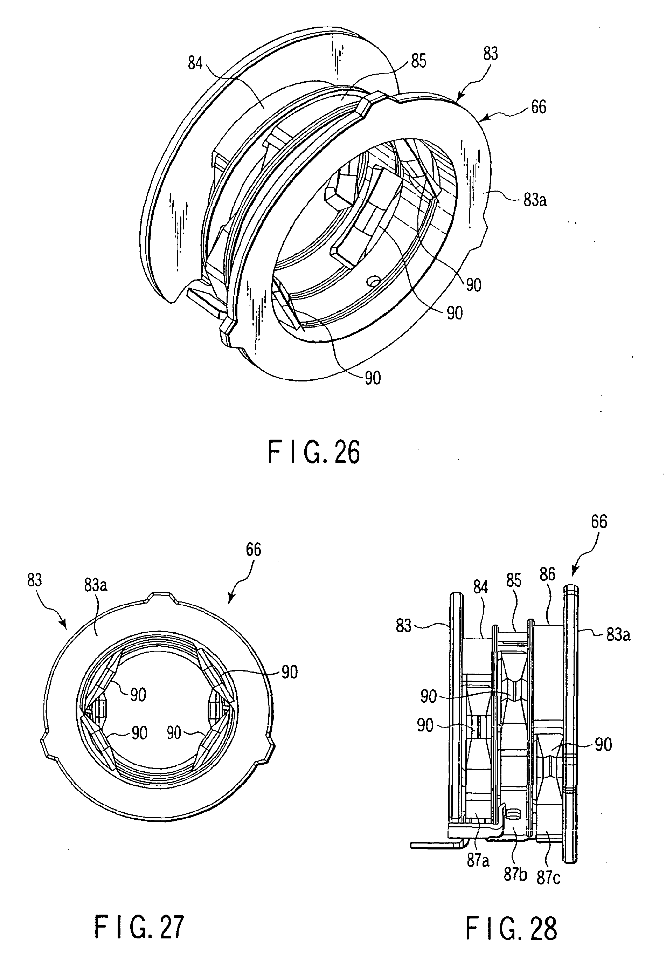

FIGS. 26 to 28 show the cylindrical contact unit 66. The contact unit 66 has a cylindrical electrode holding member 83 made of a resin. The electrode holding member 83 has three (first to third) electrode receiving portions 84, 85 and 86 different in the size of outside diameter, as shown in FIG. 28. The first electrode receiving portion 84 on the distal end side has the smallest diameter, and the third electrode receiving portion 86 on the rear end side has the largest diameter.

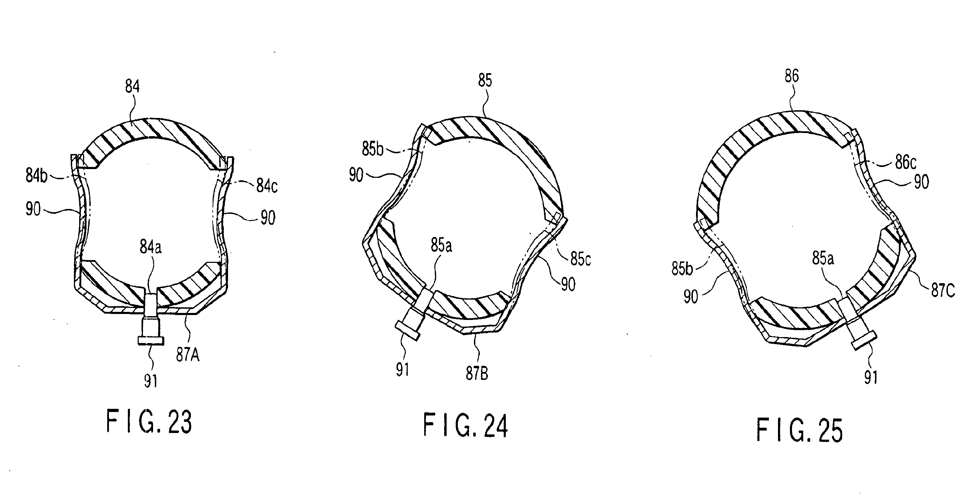

As shown in FIG. 23, the first electrode receiving portion 84 has one contact member fixing hole 84a and two through-holes 84b and 84c. The central lines of the two through-holes 84b and 84c are disposed at positions perpendicular to the central line of the contact member fixing hole 84a.

In the same manner, the second electrode receiving portion 85 has one contact member fixing hole 85a and two through-holes 85b and 85c, as shown in FIG. 24. The third electrode receiving portion 86 has one contact member fixing hole 86a and two through-holes 86b and 86c, as shown in FIG. 25.

The contact member fixing hole 84a of the first electrode receiving portion 84, the contact member fixing hole 85a of the second electrode receiving portion 85 and the contact member fixing hole 86a of the third electrode receiving portion 86 are positioned so that they are displaced from each other in the circumferential direction of the electrode holding member 83.

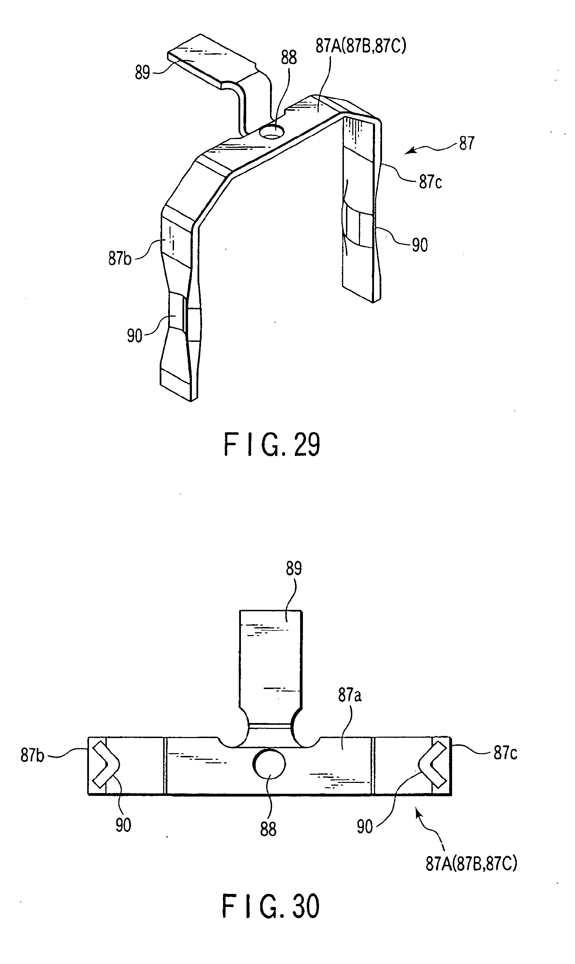

FIGS. 29 and 30 show electrode members 87A, 87B and 87C to be set to the first to third electrode receiving portions 84, 85 and 86. These electrode members 87A, 87B and 87C are formed to have the same shape. Here, the electrode member 87A to be set to the first electrode receiving portion 84 alone will be described, and the same signs are assigned to the same parts of the other electrode members 87B and 87C of the second and third electrode receiving portions 85 and 86, so that the electrode members 87B and 87C will not be described.

The electrode member 87A has one linear fixed portion 87a, and two bending portions 87b and 87c. The one bending portion 87b is disposed at one end of the linear fixed portion 87a, and the other bending portion 87c is disposed at the other end thereof. Thus, the electrode member 87A is formed to be bent into a substantially U shape, as shown in FIG. 29.

A hole 88 and an L-shaped wiring line connecting portion 89 are provided at the central position of the fixed portion 87a. Constricted portions 90 having an inwardly curving shape are formed in the two bending portions 87b and 87c at their central positions.

When the electrode member 87A is set to the first electrode receiving portion 84, a fixing pin 91 is inserted into the hole 88 of the fixed portion 87a of the electrode member 87A and into the contact member fixing hole 85a of the first electrode receiving portion 84. The electrode member 87A is fixed to the first electrode receiving portion 84 by the fixing pin 91. At this point, the constricted portion 90 of the one bending portion 87b of the electrode member 87A is disposed to be inserted into the one through-hole 85b of the first electrode receiving portion 84, while the constricted portion 90 of the other bending portion 87c of the electrode member 87A is disposed to be inserted into the other through-hole 85c. The same holds true for the case where the electrode member 87B is set to the second electrode receiving portion 85 and for the case where the electrode member 87C is set to the third electrode receiving portion 86.

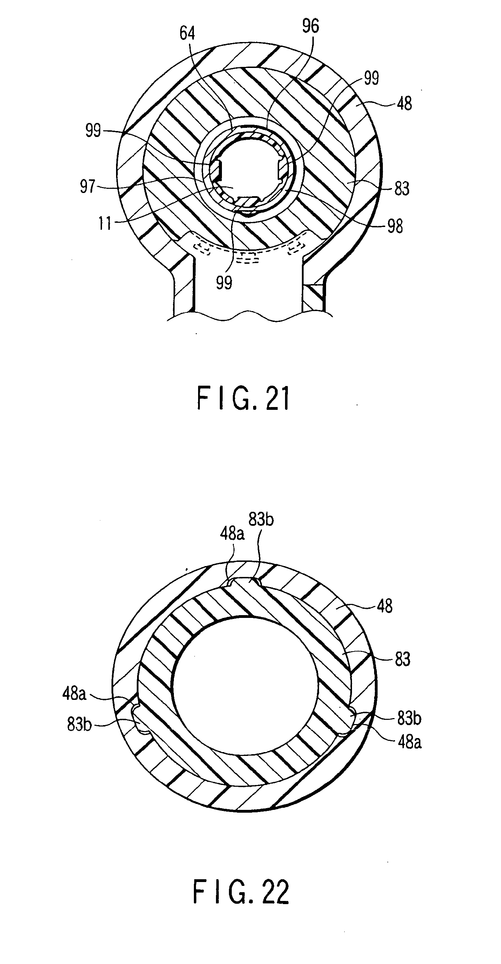

As shown in FIG. 22, a large-diameter fixed flange portion 83a is formed at the rear end of the electrode holding member 83 of the contact unit 66. Engaging convex portions 83b are provided to protrude on the outer peripheral surface of the fixed flange portion 83a at a plurality of places, in the present embodiment, at three places. Engaging concave portions 48a are formed on the inner peripheral surface at the rear end of the holding cylinder 48 at positions corresponding to the three engaging convex portions 83b of the fixed flange portion 83a. When the electrode holding member 83 is set to the holding cylinder 48, they are engaged with and fixed to each other so that the three engaging convex portions 83b of the fixed flange portion 83a are inserted into the engaging concave portions 48a of the holding cylinder 48. This regulates the rotation of the electrode holding member 83 with respect to the holding cylinder 48 in the direction around the axis.

A step portion 43b for contacting the fixed flange portion 83a of the electrode holding member 83 is formed in the holding cylinder 48. The electrode holding member 83 is screwed to the holding cylinder 48 by a fixing screw 48c so that the fixed flange portion 83a of the electrode holding member 83 is placed in collision with this step portion 43b. This regulates the axial movement of the electrode holding member 83 with respect to the holding cylinder 48.

The ends of three wiring lines 93a to 93c incorporated in the switch holding portion 51 are connected to the wiring line connecting portions 89 of the three electrode members 87A, 87B and 87C set to the contact unit 66.

The contact unit 66 is further provided with a substantially C-shaped electric contact member 96 configured by a metal leaf spring, as shown in FIG. 21. The electric contact member 96 is connected to the outer peripheral surface at the proximal end of the spring bearing member 64.

The handle unit side electric path 95 comprises the electric contact member 96, the spring bearing member 64, the positioning pins 81 and the rotation transmitting member 71.

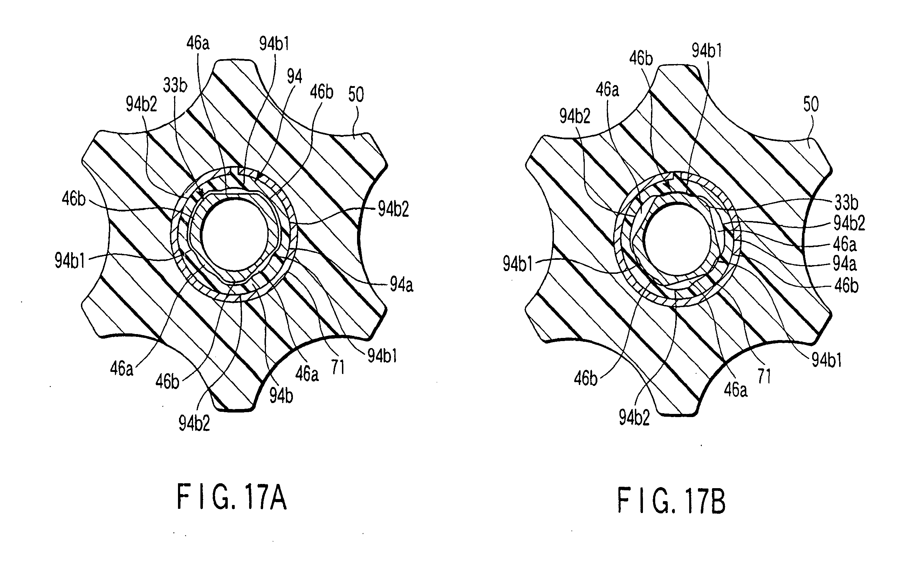

On the inner peripheral surface of the rotation transmitting member 71, there is provided engaging means 94 for removably engaging with the outer peripheral flange portion 33b of the sheath unit 5 substantially at the central position along the axial direction. As shown in FIGS. 17A and 17B, this engaging means 94 has an insertion hole 94a into which the outer peripheral flange portion 33b is inserted when the sheath unit 5 is coupled to the handle unit 4, and a conductive rubber ring (urging means) 94b disposed in the insertion hole 94a.

The shape of the inner peripheral surface of the conductive rubber ring 94b is substantially the same as that of the engaging portion 46 of the outer peripheral flange portion 33b. In other words, there are formed three plane portions 94b1 cut at a plurality of places, in the present embodiment, at three places on the circular inner peripheral surface, and three corner portions 94b2 which are disposed at junctions between the three plane portions 94b1 and which have diameters larger than those of the plane portions 94b1. This forms a sectional shape substantially close to a triangular shape. Therefore, the conductive rubber ring 94b is held at a non-compression position where it is in a natural state, at a position where the shape of the inner peripheral surface of the conductive rubber ring 94b corresponds to the engaging portion 46 of the outer peripheral flange portion 33b, that is, in a situation where the three corner portions 46b of the outer peripheral flange portion 33b correspond to the three corner portions 94b2 of the conductive rubber ring 94b, as shown in FIG. 17A. On the contrary, if the handle unit 4 and the sheath unit 5 are rotated relatively to each other in the direction around the central axis of the sheath unit 5, the conductive rubber ring 94b is switched to a pressure-contact position at which the conductive rubber ring 94b is brought into pressure-contact with the three corner portions 46b of the outer peripheral flange portion 33b, as shown in FIG. 17B. At this point, the three corner portions 46b of the outer peripheral flange portion 33b contact the three plane portions 94b1 of the conductive rubber ring 94b, and are thus compressed.

In the present embodiment, the conductive rubber ring 94b is held at the non-compression position where it is in the natural state as shown in FIG. 17A during an insertion operation (see FIGS. 31 and 32) in which the outer peripheral flange portion 33b of the sheath unit 5 is inserted straight into the conductive rubber ring 94b when the sheath unit 5 is coupled to the handle unit 4. At this point, the engaging lever 43 on the side of the handle unit 4 is held while being stranded on the inclined surface of the guide groove 41 of the pinch member 32 of the sheath unit 5. Then, the pinch member 32 of the sheath unit 5 is rotated with respect to the handle unit 4 in a direction around the axis, such that the engaging lever 43 on the side of the handle unit 4 engages in an inserted state with the engaging concave portion 42 at one end of the guide groove 41, as shown in FIGS. 33 and 34. At this point, the conductive rubber ring 94b is switched to a pressure-contact position at which the conductive rubber ring 94b is brought into pressure-contact with the three corner portions 46b of the outer peripheral flange portion 33b, as shown in FIG. 17B. This permits conduction, via the conductive rubber ring 94b, between the sheath unit side electric path 40 (formed between the guide cylindrical member 33, the fixing screw 39, the joining pipe 38, the outer cylinder 18, the distal end cover 25, the supporting point pins 27 and the jaw main unit 28) and the handle unit side electric path 95 (formed between the electric contact member 96, the spring bearing member 64, the positioning pins 81 and the rotation transmitting member 71). At this point, a second high-frequency electric path 97 for transmitting a high-frequency current is formed in a combination of the sheath unit 5 and the handle unit 4.

As shown in FIG. 21, the handle unit 4 has a tubular member 98 formed by an insulating material on the inner peripheral surface of the spring bearing member 64. The tubular member 98 is fixed to the inner peripheral surface of the spring bearing member 64. Thus, the tubular member 98 provides insulation between the first high-frequency electric path 13 and the second high-frequency electric path 97 when the probe unit 3 is connected to the handle unit 4.

On the inner peripheral surface of the tubular member 98, there are formed three engaging convex portions 99 corresponding to the three engaging concave portions 15 (see FIG. 37) of the flange portion 14 of the probe unit 3. When the probe unit 3 is connected to the handle unit 4, the three engaging convex portions 99 of the tubular member 98 removably engage with the three engaging concave portions 15 of the flange portion 14 of the probe unit 3. This regulates the positions of the probe unit 3 and the tubular member 98 of the handle unit 4 in the rotation direction. Thus, a combination of the probe unit 3 and the transducer unit 2 is driven to integrally rotate together with a set unit inside the holding cylinder 48 during the rotational operation of the swing operation knob 50.

In addition, the engaging portion between the flange portion 14 of the probe unit 3 and the tubular member 98 is not limited to the configuration described above. For example, the tubular member 98 may be formed to have a D-shaped section, and the flange portion 14 of the probe unit 3 may be formed to have a D-shaped section correspondingly.

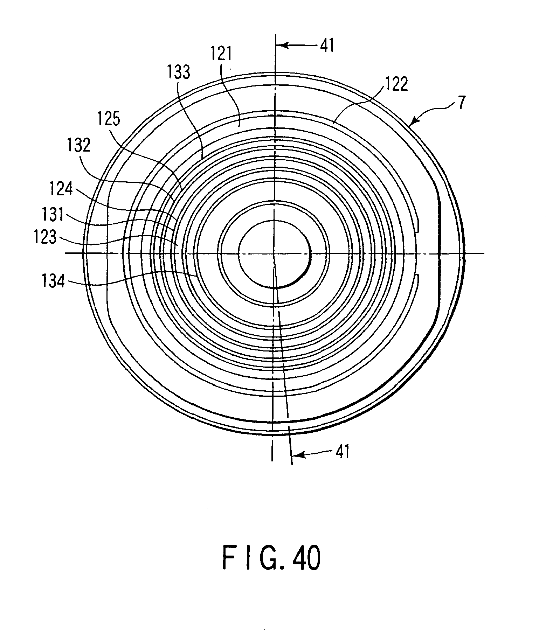

The front end of the transducer unit 2 is removably coupled to the contact unit 66. In one cable 9 at the rear end of the transducer unit 2, there are incorporated two wiring lines 101 and 102 for the ultrasonic transducer, two wiring lines 103 and 104 for high-frequency conduction, and three wiring lines 105, 106 and 107 connected to the wiring line circuit board 503a within the switch holding portion 51, as shown in FIG. 40. The distal ends of the two wiring lines 101 and 102 for the ultrasonic transducer are connected to the ultrasonic transducer 6. The distal end of the one wiring line 103 for the high-frequency conduction is connected to the ultrasonic transducer 6.

Four first to fourth conducting plates 111 to 114 for electric connection are disposed at the rear end of the transducer unit 2. The distal end of the other wiring line 104 for high-frequency conduction is connected to the first conducting plate 111. The three wiring lines 105, 106 and 107 are connected to the second to fourth conducting plates 112 to 114, respectively.

FIG. 41 shows an internal configuration of the front end of the transducer unit 2. A connection cylindrical portion 121 is formed at the distal end of the transducer cover 7. A leaf-spring-shaped C ring 122 in which a part of a ring is cut off is attached onto the outer peripheral surface of the connection cylindrical portion 121. Three steps of (first to third) cylindrical portions 123 to 125 which have differently dimensioned outside diameters are provided to protrude inside the connection cylindrical portion 121. The first cylindrical portion 123 has the smallest outside diameter, and the largest length of protrusion from the distal end of the connection cylindrical portion 121. The second cylindrical portion 124 has an outside diameter larger than that of the first cylindrical portion 123, and the length of its protrusion from the distal end of the connection cylindrical portion 121 is smaller than that of the first cylindrical portion 123. The third cylindrical portion 125 has the largest outside diameter, and the length of its protrusion from the distal end of the connection cylindrical portion 121 is smaller than that of the second cylindrical portion 124.

A cylindrical first contact member 131 is attached onto the outer peripheral surface of the first cylindrical portion 123. In the same manner, a cylindrical second contact member 132 is attached onto the outer peripheral surface of the second cylindrical portion 124, and a cylindrical third contact member 133 is attached onto the outer peripheral surface of the third cylindrical portion 125. The second conducting plate 112 is connected to the first contact member 131, the third conducting plate 113 is connected to the second contact member 132, and the fourth conducting plate 114 is connected to the third contact member 133.

A cylindrical fourth contact member 134 is attached onto the inner peripheral surface of the first cylindrical portion 123. The fourth contact member 134 is connected to the first conducting plate 111.

When the handle unit 4 is coupled to the transducer unit 2, the contact unit 66 of the handle unit 4 is connected to the front end of the transducer unit 2. At this point, the electrode member 87A of the contact unit 66 is connected to the first contact member 131 of the transducer unit 2. At the same time, the electrode member 87B of the contact unit 66 is connected to the second contact member 132 of the transducer unit 2, the electrode member 87C of the contact unit 66 is connected to the third contact member 133 of the transducer unit 2, and the C-shaped electric contact member 96 of the contact unit 66 is connected to the fourth contact member 134 of the transducer unit 2.

Next, effects of the present embodiment will be described. In the hand piece 1 of the ultrasonic operating apparatus of the present embodiment, the four units including the transducer unit 2, the probe unit 3, the handle unit 4 and the sheath unit 5 are detachable, as shown in FIG. 2. During the use of the hand piece 1, the transducer unit 2 is coupled to the probe unit 3. Thus, the first high-frequency electric path 13 for transmitting the high-frequency current is formed in the combination of the transducer unit 2 and the probe unit 3.

Subsequently, the handle unit 4 is coupled to the sheath unit 5. When the handle unit 4 is coupled to the sheath unit 5, the connecting pipe member 34 is inserted into the rotation transmitting member 71 of the handle unit 4 while the pinch member 32 of the sheath unit 5 is being gripped. When the sheath unit 5 is coupled to the handle unit 4, the engaging lever 43 on the side of the handle unit 4 is held while being stranded on the inclined surface of the guide groove 41 of the pinch member 32 of the sheath unit 5, as shown in FIGS. 31 and 32. At this point, as shown in FIG. 17A, the engaging lever 43 is held at the position where the shape of the inner peripheral surface of the conductive rubber ring 94b corresponds to the engaging portion 46 of the outer peripheral flange portion 33b, that is, in a situation where the three corner portions 46b of the outer peripheral flange portion 33b correspond to the three corner portions 94b2 of the conductive rubber ring 94b. Therefore, the outer peripheral flange portion 33b of the sheath unit 5 is inserted straight into the conductive rubber ring 94b. During this insertion operation, the conductive rubber ring 94b is held at the non-compression position where it is in the natural state, as shown in FIG. 17A. In this state, there is no conduction between the sheath unit side electric path 40 and the handle unit side electric path 95.

Then, after this insertion operation is finished, the pinch member 32 of the sheath unit 5 is rotated in the direction around the axis with respect to the handle unit 4. Owing to this operation, the engaging lever 43 on the side of the handle unit 4 engages in an inserted state with the engaging concave portion 42 at one end of the guide groove 41, as shown in FIGS. 33 and 34. At this point, the conductive rubber ring 94b is switched to the pressure-contact position at which the conductive rubber ring 94b is placed in pressure-contact with the three corner portions 46b of the outer peripheral flange portion 33b, as shown in FIG. 17B. This permits conduction, via the conductive rubber ring 94b, between the sheath unit side electric path 40 and the handle unit side electric path 95. As a result, the second high-frequency electric path 97 for transmitting a high-frequency current is formed in the combination of the sheath unit 5 and the handle unit 4.

During this rotational operation of the sheath unit 5 in a direction around the axis, the pair of engaging pins 45 on the side of the handle unit 4 removably engages with the engaging grooves 44a at the terminal ends of the guide grooves 44 of the sheath unit 5 at the same time. Thus, the spring bearing member 64 on the side of the handle unit 4 is coupled to the connecting pipe member 34 on the side of the sheath unit 5 via the engaging pins 45. As a result, the operation force on the side of the handle unit 4 during the operation of closing the movable handle 49 with respect to the fixed handle 47 can be transmitted to the drive shaft 21 of the jaw 17 on the side of the sheath unit 5. This is the state where the sheath unit 5 is coupled to the handle unit 4.

Subsequently, the combination of the sheath unit 5 and the handle unit 4 and the combination of the ultrasonic transducer 6 and the probe unit 3 are set to be united into one. During this setting operation, the contact unit 66 of the handle unit 4 is connected to the front end of the transducer unit 2. At this point, the electrode member 87A of the contact unit 66 is connected to the first contact member 131 of the transducer unit 2. At the same time, the electrode member 87B of the contact unit 66 is connected to the second contact member 132 of the transducer unit 2, the electrode member 87C of the contact unit 66 is connected to the third contact member 133 of the transducer unit 2, and the C-shaped electric contact member 96 of the contact unit 66 is connected to the fourth contact member 134 of the transducer unit 2. Thus, the second high-frequency electric path 97 of the combination of the sheath unit 5 and the handle unit 4 is connected to the wiring line 104 for the high-frequency conduction within the cable 9. Further, the three wiring lines 105, 106 and 107 within the cable 9 are connected to the wiring line circuit board 503a within the switch holding portion 51. This is the state where the setting of the hand piece 1 is finished.

Then, during the use of this hand piece 1, the thumb H1 is inserted into the thumb insertion ring portion 62 of the movable handle 49, and the plurality of fingers H3, H4 and H5 except for the thumb H1 and index finger H2 are inserted into the multiple finger insertion ring portion 61 of the fixed handle 47, as shown in FIG. 52, such that the hand piece 1 is gripped. At this point, when the two switches (the first switch 54 and the second switch 55) of the switch unit 503 are not operated, the index finger H2 is held in touch with the bulging portion 501 of the switch attachment surface 52a. In this state, the movable handle 49 is closed with respect to the fixed handle 47. The drive shaft 21 is axially moved in conjunction with the operation of this movable handle 49, and the jaw 17 is driven to open/close with respect to the probe distal end 3a of the probe unit 3 in conjunction with the axial back-and-forth movement of the drive shaft 21. Thus, the living tissue is gripped between the jaw 17 and the probe distal end 3a of the probe unit 3.

In this state, one of the first switch button 54a and the second switch button 55a of the movable handle 49 is selectively pushed. When the second switch button 55a is pushed, electricity is conducted in the first high-frequency electric path 13 for conducting a high-frequency current to the probe distal end 3a of the probe unit 3 and in the second high-frequency electric path 97 for conducting a high-frequency current to the jaw main unit 28 of the sheath unit 5. Thus, two bipolar electrodes for the high-frequency treatment are formed by the probe distal end 3a of the probe unit 3 and the jaw main unit 28 of the sheath unit 5. Then, the high-frequency current is conducted across the two bipolar electrodes formed by the probe distal end 3a of the probe unit 3 and the jaw main unit 28 of the sheath unit 5, such that the living tissue between the jaw 17 and the probe distal end 3a of the probe unit 3 can be subjected to the high-frequency treatment by the bipolar.

When the first switch button 54a is pushed, a drive current is conducted to the ultrasonic transducer 6 simultaneously with the high frequency conduction, and the ultrasonic transducer 6 is driven. Thus, the ultrasonic vibrations from the ultrasonic transducer 6 are transmitted to the probe distal end 3a via the vibration transmitting member 11, such that the treatment such as the incision or removal of the living tissue can be administered using the ultrasonic waves simultaneously with the high frequency conduction. In addition, the ultrasonic waves can also be used to coagulate the living tissue.

Furthermore, during the rotational operation of the swing operation knob 50, the rotational operation of the rotation transmitting member 71 which rotates together with the swing operation knob 50 is transmitted to the side of the spring bearing member 64 via the pins 81. Thus, during the rotational operation of the swing operation knob 50, the set unit of the rotation transmitting member 71, the pins 81, the spring bearing member 64, the slider member 65 and the coil spring 67 within the holding cylinder 48 are driven to integrally rotate in a direction around the axis together with the swing operation knob 50. Moreover, the rotational operation force of the swing operation knob 50 is transmitted to the vibration transmitting member 11 of the probe unit 3 via the tubular member 98 which rotates together with the spring bearing member 64 within the holding cylinder 48. Thus, the set unit within the holding cylinder 48 and the combination of the transducer unit 2 and the probe unit 3 are driven to integrally rotate together in a direction around the axis.

Therefore, the configuration described above provides the following advantages: the first switch 54 and the second switch 55 are vertically arranged in the switch holding portion 51 between the fixed handle 47 and the holding cylinder 48 in the hand piece 1 of the ultrasonic treatment apparatus in the present embodiment. Moreover, the bulging portion 501 is disposed between the first switch 54 and the second switch 55. Therefore, when the switch 54 or 55 is operated with the index finger H2 of the user gripping the handle unit 4, the position of the first switch 54 can be distinguished from the position of the second switch 55 on the basis of the position of the bulging portion 501. This ensures that the user can differentiate between the first switch 54 and the second switch 55 that have different functions.

Furthermore, the bulging portion 501 is set so that the height of projection of this bulging portion from the switch attachment surface 52a is larger than the height of projection of the first switch 54 and the second switch 55 from the attachment surface 52a. Therefore, the user gripping the handle unit 4 can easily distinguish between the bulging portion 501 and the first and second switches 54 and 55 in accordance with the feeling in the index finger H2 touching the bulging portion 501 and the first and second switches 54 and 55. This can omit the visual identification of the first switch 54 and the second switch 55 and therefore provides an advantage that the user gripping the handle unit 4 is allowed to easily operate the first switch 54 and the second switch 55.

Still further, the bulging portion 501 has the extension 502 which continuously extends from the switch attachment surface 52a of the fixed handle 47 over both lateral sides thereof. Therefore, except for the case where the index finger H2 of the user operates the first switch 54 and the second switch 55 from the front side of the switch attachment surface 52a, the index finger H2 of the user can touch the extension 502 of the bulging portion 501 to easily distinguish the first switch 54 from the second switch 55 even if the index finger H2 of the user operates the first switch 54 and the second switch 55 from the side surface of the switch attachment surface 52a.

Still further, in the present embodiment, the switch attachment surface 52a has the curving surface 506 curving along the flow line L1 on which the index finger H2 moves in a condition where the thumb H1 is inserted into the thumb H1 insertion ring portion 62 and the plurality of fingers H3, H4 and H5 except for the thumb H1 and index finger H2 are inserted into the multiple finger insertion ring portion 61 as shown in FIG. 52. Further, the switch unit 503 is attached to the unit receiver 504 so that the base member 503c curves along the curving surface 506. Thus, the first switch 54 and the second switch 55 can be arranged at positions when they can be easily pushed by the user with the index finger H2. This can reduce fatigue from the switch operation as compared with the case where the switches are positioned immediately above the middle finger. It is also possible to prevent the movement of other fingers following the movement of the index finger H2 when the switches 54 and 55 are operated with the index finger H2.

Further yet, in the present embodiment, the unit receiver 504 has the two bosses 505a and 505b for receiving the force to push the push buttons 54a and 55a for the two switches, as shown in FIG. 16B. Then, the force to push the push button 54a for the first switch 54 is received by the boss 505a, and the force to push the push button 55a for the second switch 55 is received by the boss 505b. This can stabilize the operation of the flexible switch unit 503.

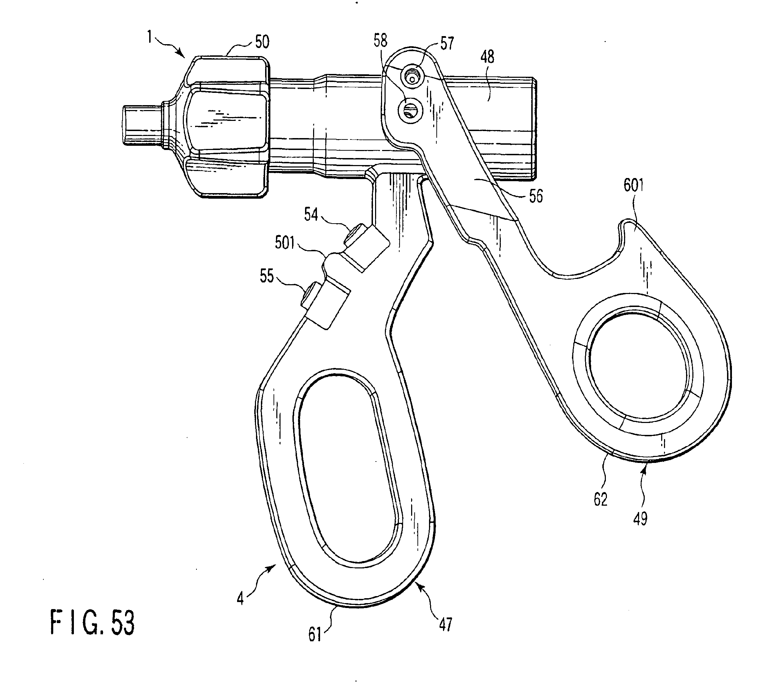

FIG. 53 shows the configuration of essential parts of a hand piece 1 of an ultrasonic treatment apparatus in a second embodiment of the present invention. A movable handle 49 has a finger hook 601 upwardly protruding on the top of a thumb insertion ring portion 62.

In this configuration, during the use of this hand piece 1, the movable handle 49 can be operated so that the thumb H1 of the user is hooked on the finger hook 601 on the top of the thumb insertion ring portion 62. This makes it possible to adapt to the use of many users.

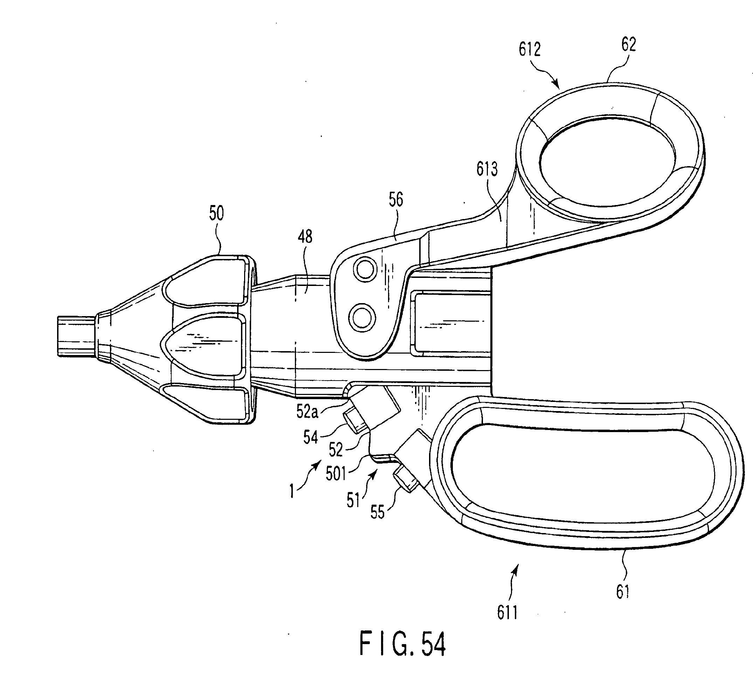

FIG. 54 shows an ultrasonic treatment apparatus in a third embodiment of the present invention. In the present embodiment, the configuration of the hand piece 1 of the ultrasonic treatment apparatus in the first embodiment (see FIGS. 1 to 52) is modified in the following manner.

That is, in a hand piece 1 in the present embodiment, a fixed handle (fixed handle element) 611 is fixed onto one side of a holding cylinder 48. Moreover, a movable handle (movable handle element) 612 is disposed on the other side of the holding cylinder 48, that is, on the side opposite to the side where the fixed handle 611 is fixed.

A multiple finger insertion ring portion 61 of the fixed handle 611 is provided to extend backward from the one side of the holding cylinder 48 along the long axis direction of a probe unit 3. A switch holding portion 51 having about the same configuration as that in the first embodiment is disposed between the holding cylinder 48 and the multiple finger insertion ring portion 61. A switch attachment surface 52a is provided on the front side of a switch attachment portion 52 of the switch holding portion 51. A first switch 54 and a second switch 55 are arranged on the switch attachment surface 52a. Moreover, on the switch attachment surface 52a, a bulging portion 501 is disposed between the first switch 54 and the second switch 55. The bulging portion 501 divides the switches 54 and 55, and doubles as a finger receiving portion.

In the movable handle 612, one end of a bending arm 613 bending perpendicularly to the U-shaped portion of a U-shaped arm 56 is coupled to the base of this arm 56. The other end of the bending arm 613 extends toward the rear of the hand piece 1. The thumb insertion ring portion 62 is formed at the extending end of this bending arm 613. Other parts are configured in the same manner as those in the first embodiment.

Thus, this configuration provides the following advantages: in the hand piece 1 of the ultrasonic treatment apparatus in the present embodiment, the switch holding portion 51 having about the same configuration as that in the first embodiment is disposed between the multiple finger insertion ring portion 61 of the fixed handle 611 and one side of the holding cylinder 48. Thus, the present embodiment also provides the same effects as the effects in the first embodiment.

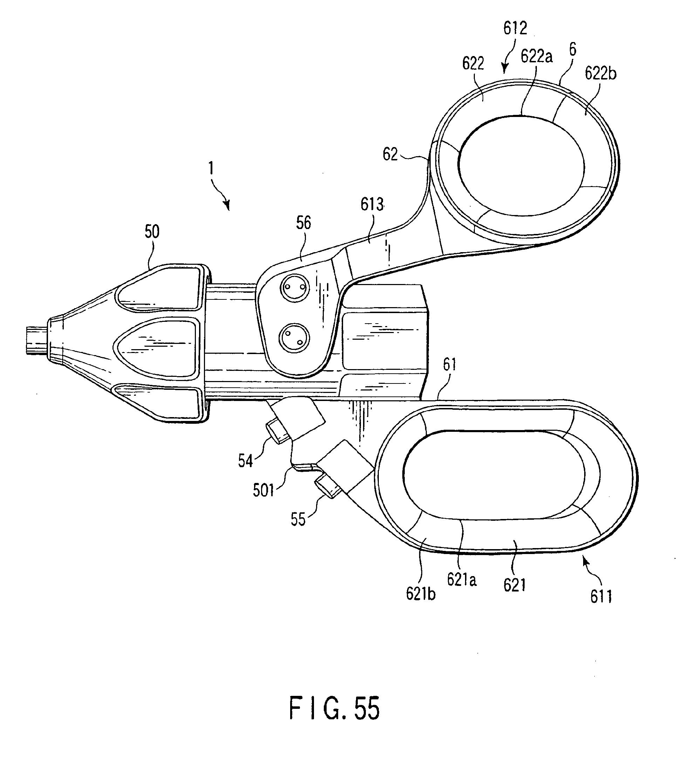

FIG. 55 shows an ultrasonic treatment apparatus in a fourth embodiment of the present invention. In the present embodiment, the configuration of the hand piece 1 of the ultrasonic treatment apparatus in the third embodiment (see FIG. 54) is modified in the following manner.

That is, in a hand piece 1 in the present embodiment, a finger pad portion 621 made of an elastic material is detachably attached to a multiple finger insertion ring portion 61 of a fixed handle 611. This finger pad portion 621 is formed into the same shape as the shape of the inner peripheral surface of the multiple finger insertion ring portion 61. In this finger pad portion 621, there are formed an inner peripheral surface cover 621a covering the inner peripheral surface of the multiple finger insertion ring portion 61, and two side covers 621b provided to extend on both sides of the inner peripheral surface cover 621a. Thus, when the finger pad portion 621 is attached to the multiple finger insertion ring portion 61, the inner peripheral surface cover 621a of the finger pad portion 621 covers the inner peripheral surface of the multiple finger insertion ring portion 61, and the two side covers 621b of the finger pad portion 621 cover the both side surfaces of the multiple finger insertion ring portion 61.

Furthermore, a finger pad portion 622 also made of an elastic material is detachably attached to a thumb insertion ring portion 62 of a movable handle 612. This finger pad portion 622 is formed into the same shape as the shape of the inner peripheral surface of the thumb insertion ring portion 62. In this finger pad portion 622, there are formed an inner peripheral surface cover 622a covering the inner peripheral surface of the thumb insertion ring portion 62, and two side covers 622b provided to extend on both sides of the inner peripheral surface cover 622a. Thus, when the finger pad portion 622 is attached to the thumb insertion ring portion 62, the inner peripheral surface cover 622a of the finger pad portion 622 covers the inner peripheral surface of the thumb insertion ring portion 62, and the two side covers 622b of the finger pad portion 622 cover the both side surfaces of the thumb insertion ring portion 62.

Thus, the configuration described above provides the following advantages: in the hand piece 1 of the ultrasonic treatment apparatus in the present embodiment, the finger pad portion 621 formed of an elastic material is detachably attached to the multiple finger insertion ring portion 61 of the fixed handle 611. Moreover, the finger pad portion 622 also formed of an elastic material is detachably attached to the thumb insertion ring portion 62 of the movable handle 612. Thus, in the present embodiment, a metal material is not directly touched by the plurality of fingers H3, H4 and H5 (except for the thumb H1 and index finger H2) inserted in the multiple finger insertion ring portion 61 of the fixed handle 611 and by the thumb H1 inserted in the thumb insertion ring portion 62 of the movable handle 612. This can reduce user fatigue.

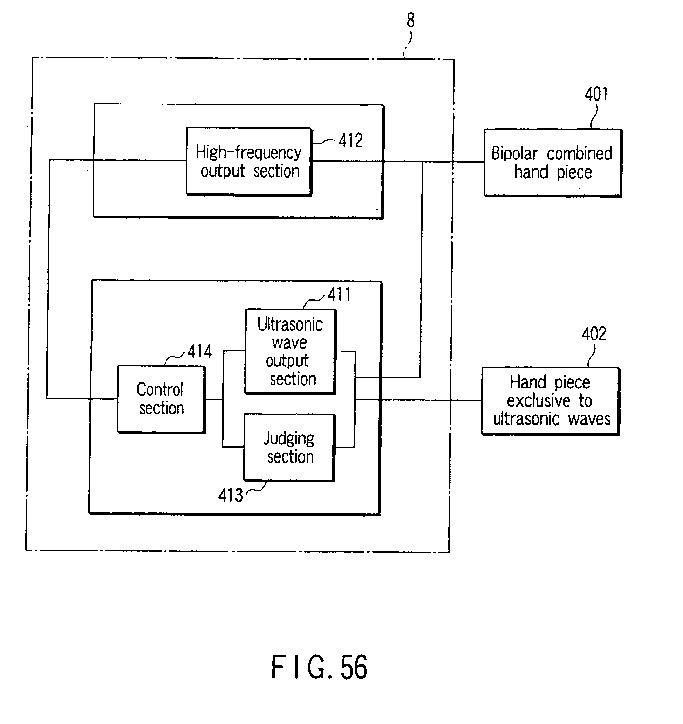

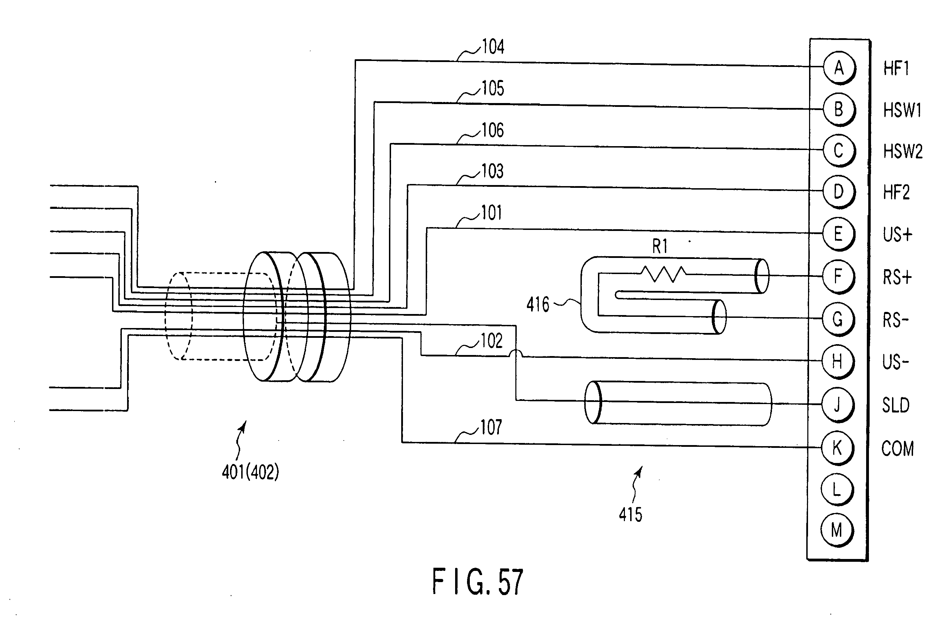

Furthermore, FIGS. 56 and 57 show a fifth embodiment of an ultrasonic treatment apparatus of the present invention. In the configuration of the present embodiment, the function of a hand switch of a fixed handle 47 is automatically switched depending on the kind of a hand piece 1 connected to a power supply main unit 8 of the ultrasonic treatment apparatus. It is to be noted that the same signs are assigned to the same parts in FIGS. 56 and 57 as those in the first embodiment, and those parts will not be described.

That is, in the present embodiment, there are connected, to the power supply main unit 8, a first hand piece 401 (corresponding to the hand piece 1 in the first embodiment) capable of the bipolar high-frequency treatment and ultrasonic treatment, and a second hand piece 402 exclusive to the ultrasonic treatment, as shown in FIG. 56.

The power supply main unit 8 has an ultrasonic wave output section 411, a high-frequency output section 412, a judging section 413 and a control section 414. The ultrasonic wave output section 411, the high-frequency output section 412 and the judging section 413 are connected to the control section 414.

FIG. 57 shows internal electric wiring lines of a connector portion 415 provided in a cable 9 of the hand piece 401, 402. Inside the connector portion 415, there is provided a model setting resistor 416 set to a different resistance value depending on the kind of the hand pieces 401 and 402.

When the connector portion 415 of the cable 9 of the hand piece 401, 402 is connected to the power supply main unit 8, the resistance value of the resistor 416 is detected by the judging section 413 of the power supply main unit 8. Then, the model of the hand piece 401, 402 connected to the power supply main unit 8 is judged in accordance with the detected resistance.

Data on the model of the hand piece 401, 402 judged by the judging section 413 is output to the control section 414. This control section 414 automatically switches the function of the hand switch of the fixed handle 47 depending on the model of the hand piece 401, 402. That is, when the first hand piece 401 is connected to the power supply main unit 8, a first switch 54a functions as an on/off switch for the bipolar high-frequency treatment, and a second switch 55a functions as an on/off switch for a combination of the ultrasonic treatment and the bipolar high-frequency treatment.

On the other hand, when the second hand piece 402 is connected to the power supply main unit 8, the first switch 54a functions as an on/off switch for driving an ultrasonic transducer 6 under a condition where its output is set, and the second switch 55a functions as an on/off switch for driving the ultrasonic transducer 6 under a condition where its output is high.

Therefore, the configuration described above provides the following advantages: in the present embodiment, the function of the hand switch of the fixed handle 47 can be automatically switched depending on the kind of the hand piece 1 connected to the power supply main unit 8 of the ultrasonic operating apparatus. There is thus no need for troublesome tasks of, for example, changing the setting of the power supply main unit 8 depending on the model of the hand piece 401, 402 connected to the power supply main unit 8 of the ultrasonic operating apparatus, and workability can be enhanced.

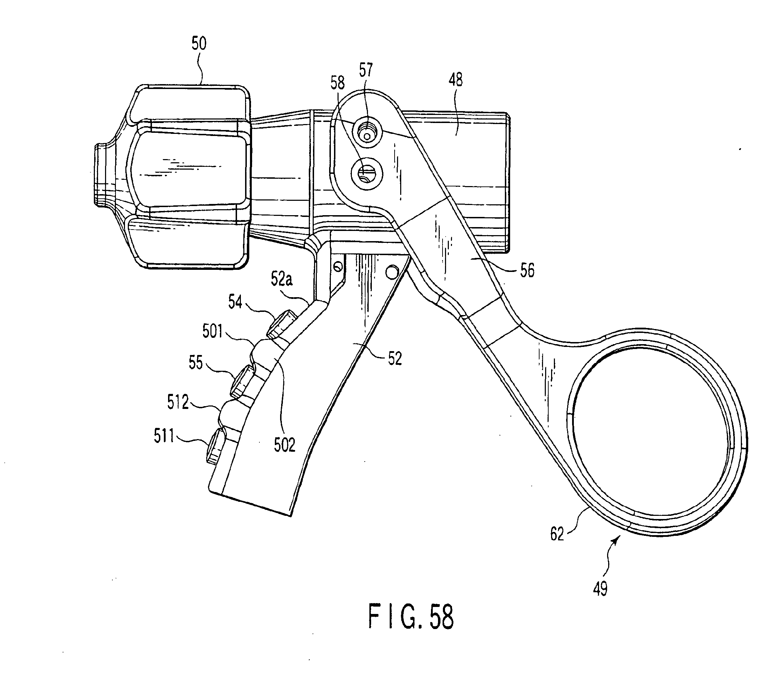

FIG. 58 shows the configuration of essential parts of an ultrasonic treatment apparatus in a sixth embodiment of the present invention. In the present embodiment, the configuration of the hand piece 1 of the ultrasonic treatment apparatus in the first embodiment (see FIGS. 1 to 52) is modified in the following manner.

That is, in a hand piece 1 in the present embodiment, three switches (a first switch 54, a second switch 55 and a third switch 511) are vertically arranged on a switch attachment surface 52a of a switch holding portion 51 of a fixed handle 47. Moreover, on the switch attachment surface 52a, a bulging portion 501 is disposed between the first switch 54 and the second switch 55. Likewise, a bulging portion 512 is disposed between the second switch 55 and the third switch 511. The bulging portion 501 divides the switches 54 and 55, and doubles as a finger receiving portion. Likewise, the bulging portion 512 divides the second switch 55 and the third switch 511, and doubles as a finger receiving portion. In addition, the shape of the bulging portion 501 may be different from the shape of the bulging portion 512. In this case, the three switches (a first switch 54, a second switch 55 and a third switch 511) can be more easily differentiated from each other.

When the first switch 54 is operated, a drive current is conducted to an ultrasonic transducer 6 simultaneously with the high frequency conduction, and the ultrasonic transducer 6 is driven. Thus, the ultrasonic vibrations from the ultrasonic transducer 6 are transmitted to a probe distal end 3a via a vibration transmitting member 11, such that the treatment such as the incision or removal of the living tissue can be administered using the ultrasonic waves simultaneously with the high frequency conduction.

When the second switch 55 is operated, the high frequency conduction alone, for example, is carried out. Thus, two bipolar electrodes for the high-frequency treatment are formed by the probe distal end 3a of the probe unit 3 and a jaw main unit 28 of a sheath unit 5. Then, the high-frequency current is conducted across the two bipolar electrodes formed by the probe distal end 3a of the probe unit 3 and the jaw main unit 28 of the sheath unit 5, such that the living tissue between the jaw 17 and the probe distal end 3a of the probe unit 3 can be subjected to the high-frequency treatment by the bipolar.

When the third switch 511 is operated, the ultrasonic transducer 6 alone, for example, is driven. Thus, the ultrasonic vibrations from the ultrasonic transducer 6 are transmitted to the probe distal end 3a via the vibration transmitting member 11, such that the treatment such as the incision or removal of the living tissue can be administered using the ultrasonic waves. In addition, the ultrasonic waves can also be used to coagulate the living tissue.

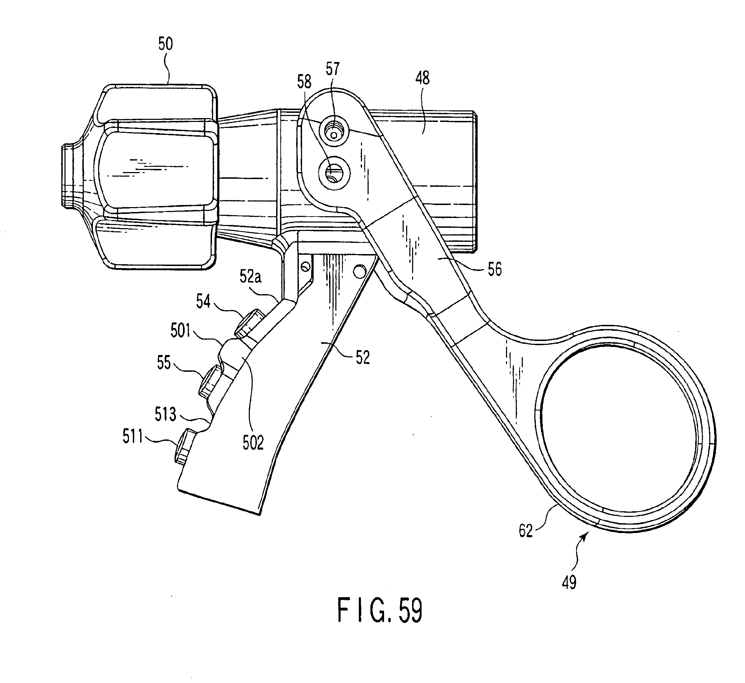

FIG. 59 shows the configuration of essential parts of an ultrasonic treatment apparatus in a seventh embodiment of the present invention. In the present embodiment, the configuration of the hand piece 1 of the ultrasonic treatment apparatus in the sixth embodiment (see FIG. 58) is modified in the following manner.

That is, in a hand piece 1 in the present embodiment, three switches (a first switch 54, a second switch 55 and a third switch 511) are vertically arranged on a switch attachment surface 52a of a switch holding portion 51 of a fixed handle 47. Moreover, on the switch attachment surface 52a, a bulging portion 501 is disposed between the first switch 54 and the second switch 55.

Furthermore, a concave portion 513 recessed in the switch attachment surface 52a is formed between the second switch 55 and the third switch 511. The bulging portion 501 divides the switches 54 and 55, and doubles as a finger receiving portion. The concave portion 513 functions as a mark for dividing the second switch 55 and the third switch 511.

Moreover, the functions of the first switch 54, the second switch 55 and the third switch 511 are similar to those in the sixth embodiment.

It is to be noted that the present invention is not limited to the embodiments described above.

## Claims

1. A surgical operating apparatus comprising:
an insertion portion (5) which includes a distal end and a proximal end and which includes a long axis and which is configured to be inserted into a body;
a treatment portion (1A) which is disposed at the distal end of the insertion portion (5) and which has a plurality of selectable surgical functions;
an operation portion (4) disposed at the proximal end of the insertion portion (5), the operation portion (4) presenting a front side and two lateral sides;
a plurality of switches (54, 55) which are provided in the operation portion (4) and which are configured to select the surgical functions; and
a bulging portion (501) which is disposed between the switches (54, 55) and which divides the switches (54, 55) and which doubles as a finger receiving portion, wherein the operation portion (4) has, on the front side of the operation portion (4), a switch attachment surface (52a) onto which the plurality of switches (54, 55) are attached, and
the plurality of switches (54, 55) are arranged in a vertical direction of the switch attachment surface (52a),
**characterized in that** the bulging portion (501) includes an extension (502) which continuously extends from the switch attachment surface (52a) of the operation portion (4) over at least one of the two lateral sides of the operation portion (4).

2. The apparatus according to claim 1, **characterized in that**
the bulging portion (501) is set so that the height of projection of this bulging portion from the switch attachment surface (52a) is larger than the height of projection of the plurality of switches (54, 55) from the attachment surface (52a).

3. The apparatus according to claim 1, **characterized in that**
the operation portion (4) has a main body of this operation portion (4) and two handle elements (47, 49) to operate the treatment portion,
the two handle elements (47, 49) have a fixed handle element (47) fixed to the main body of the operation portion (4) and extending on a lateral side of the long axis, and a movable handle element (49) supported to be openable/closable with respect to the fixed handle element (47), and
the attachment surface (52a) is provided at a junction between the main body of the operation portion (4) and the fixed handle element (47).

4. The apparatus according to claim 3, **characterized in that**
the movable handle element (49) has a thumb insertion ring portion (62) which permits the insertion of a thumb,
the fixed handle element (47) has a multiple finger insertion ring portion (61) which permits the insertion of a plurality of fingers except for the thumb and index finger,
the switch attachment surface (52a) has a curving surface (506) curving along a flow line (L1) on which the index finger is movable in a condition where the thumb is inserted into the thumb insertion ring portion (62) and the plurality of fingers except for the thumb and index finger are inserted into the multiple finger insertion ring portion (61).

5. The apparatus according to claim 4, **characterized in that**
the operation portion (4) is set so that an angle [alpha] between a tangent line (L2) of a front surface of the multiple finger insertion ring portion (61) of the fixed handle element (47) and a tangent line (L3) of a front surface of the switch attachment surface (52a) is larger than 90[deg.].

6. The apparatus according to claim 1, **characterized in that**
the main body of the operation portion (4) has a switch unit (503), and the plurality of switches includes two switches (54, 55) which are integrated into one unit, and a concave unit receiver (504) to which the switch unit (503) is attached,
the switch unit (503) has push buttons (54a, 55a) for the two switches, a flexible wiring line circuit board (503a) for the two switches, and a flexible base member (503c) in which the wiring line circuit board (503a) is embedded in insulating elastic members, and
the base member (503c) is attached to the unit receiver (504) so that this base member curves along the curving surface (506).

7. The apparatus according to claim 6, **characterized in that**
the unit receiver (504) has, in parts corresponding to the push buttons (54a, 55a) for the two switches, boss portions (505a, 505b) which are configured to receive force to push the push buttons (54a, 55a) for the two switches.

8. The apparatus according to claim 6, **characterized in that**
the two switches (54, 55) have a first switch (54) which is disposed on the upper side of the switch attachment surface (52a) and which is configured to select a frequently used first surgical function of the plurality of surgical functions, and a second switch (55) which is disposed on the lower side of the switch attachment surface (52a) and which is configured to select another second surgical function of the plurality of surgical functions.

9. The apparatus according to claim 8, **characterized in that**
the first surgical function is a function to simultaneously output an ultrasonic treatment output and a high-frequency treatment output, and
the second surgical function is a function to independently output the high-frequency treatment output alone.

10. The apparatus according to claim 8, **characterized in that**
the first surgical function is a function to output an ultrasonic treatment output in a maximum output state, and
the second surgical function is a function to output an ultrasonic treatment output in a preset arbitrary set output state lower than the maximum output state.

11. The apparatus according to claim 4, **characterized in that**
a finger pad portion (621, 622) formed of an elastic material is detachably attached to at least one of the thumb insertion ring portion (62) and the multiple finger insertion ring portion (61).

12. The apparatus according to claim 4, **characterized in that** the movable handle element (49) has a finger hook (601) in an upper portion of the thumb insertion ring portion (62).

## Patentansprüche

1. Chirurgisches Operationsgerät, das umfasst:
einen Einführabschnitt (5), der ein distales Ende und ein proximales Ende umfasst und der eine lange Achse umfasst und der dazu eingerichtet ist, in einen Körper eingeführt zu werden;
einen Behandlungsabschnitt (1A), der an dem distalen Ende des Einführabschnitts (5) positioniert ist und der eine Mehrzahl von auswählbaren chirurgischen Funktionen hat;
einen Operationsabschnitt (4), der an dem proximalen Ende des Einführabschnitts (5) positioniert ist, wobei der Operationsabschnitt (4) eine Vorderseite und zwei laterale Seiten aufweist;
eine Mehrzahl von Schaltern (54, 55), die in dem Operationsabschnitt (4) bereitgestellt sind und die dazu eingerichtet sind, die chirurgischen Funktionen auszuwählen; und
einen wulstigen Abschnitt (501), der zwischen den Schaltern (54, 55) angeordnet ist und der die Schalter (54, 55) trennt und der auch als Fingeraufnahmeabschnitt wirkt, wobei der Operationsabschnitt (4) auf der Vorderseite des Operationsabschnitts (4) eine Schalterbefestigungsoberfläche (52a) hat, an der die Mehrzahl von Schaltern (54, 55) angebracht sind, und
die Mehrzahl von Schaltern (54, 55) in einer vertikalen Richtung der Schalterbefestigungsoberfläche (52a) angeordnet sind,
**dadurch gekennzeichnet, dass** der wulstige Abschnitt (501) eine Erweiterung (502) umfasst, die sich kontinuierlich von der Schalterbefestigungsoberfläche (52a) des Operationsabschnitts (4) über mindestens eine der zwei lateralen Seiten des Operationsabschnitts (4) erstreckt.

2. Gerät gemäß Anspruch 1, **dadurch gekennzeichnet, dass**
der wulstige Abschnitt (501) so gestaltet ist, dass die Höhe, um die der wulstige Abschnitt von der Schalterbefestigungsoberfläche (52a) vorsteht, größer als die Höhe ist, um die die Mehrzahl von Schaltern (54, 55) von der Schalterbefestigungsoberfläche (52a) vorstehen.

3. Gerät gemäß Anspruch 1, **dadurch gekennzeichnet, dass**
der Operationsabschnitt (4) einen Hauptkörper dieses Operationsabschnitts (4) und zwei Handgriffelemente (47, 49) zur Bedienung des Behandlungsabschnitts hat,
die zwei Handgriffelemente (47, 49) ein fixiertes Handgriffelement (47), das an dem Hauptkörper des Operationsabschnitts (4) fixiert ist und sich auf einer lateralen Seite der langen Achse erstreckt, und ein bewegliches Handgriffelement (49) haben, das bezüglich des fixierten Handgriffelements (47) gelagert ist, um zu öffnen/
zu schließen zu sein, und
die Befestigungsoberfläche (52a) an einer Verbindung zwischen dem Hauptkörper des Operationsabschnitts (4) und dem fixierten Handgriffelement (47) vorgesehen ist.

4. Gerät gemäß Anspruch 3, **dadurch gekennzeichnet, dass**
das bewegliche Handgriffelement (49) einen Daumeneinführringabschnitt (62) hat, der das Einführen eines Daumens erlaubt,
das fixierte Handgriffelement (47) einen Ringabschnitt (61) zum Einführen mehrerer Finger hat, der das Einführen einer Mehrzahl von Fingern mit Ausnahme des Daumens und des Zeigefingers erlaubt,
die Schalterbefestigungsoberfläche (52a) eine gekrümmte Oberfläche (506) hat, die sich entlang einer Flusslinie (L1) krümmt, auf der der Zeigefinger in einem Zustand, in dem der Daumen in den Daumeneinführringabschnitt (62) eingeführt ist und die Mehrzahl von Fingern mit Ausnahme des Daumens und Zeigefingers in den Ringabschnitt (61) zum Einführen mehrerer Finger eingeführt sind, bewegbar ist.

5. Gerät gemäß Anspruch 4, **dadurch gekennzeichnet, dass**
der Operationsabschnitt (4) so gestaltet ist, dass ein Winkel [alpha] zwischen einer Tangentenlinie (L2) einer Frontoberfläche des Ringabschnitts (61) zum Einführen mehrerer Finger des fixierten Handgriffelements (47) und einer Tangentenlinie (L3) einer Frontoberfläche der Schalterbefestigungsoberfläche (52a) größer als 90 [Grad] ist.

6. Gerät gemäß Anspruch 1, **dadurch gekennzeichnet, dass**
der Hauptkörper des Operationsabschnitts (4) eine Schaltereinheit (503) hat und die Mehrzahl von Schaltern zwei Schalter (54, 55) umfasst, die in eine Einheit integriert sind, und eine konkave Einheitenaufnahme (504) hat, an der die Schaltereinheit (503) angebracht ist,
die Schaltereinheit (503) Druckknöpfe (54a, 55a) für die zwei Schalter, eine flexible Verdrahtungslinienschaltplatte (503a) für die zwei Schalter und ein flexibles Basiselement (503c) hat, in dem die Verdrahtungslinienschaltplatte (503a) in isolierende elastische Elemente eingebettet ist, und
das Basiselement (503c) so an der Einheitenaufnahme (504) befestigt ist, dass sich dieses Basiselement entlang der gekrümmten Oberfläche (506) krümmt.

7. Gerät gemäß Anspruch 6, **dadurch gekennzeichnet, dass**
die Einheitenaufnahme (504), teilweise entsprechend den Druckknöpfen (54a, 55a) für die zwei Schalter, vorstehende Abschnitte (505a, 505b) hat, die dazu eingerichtet sind, eine Kraft zum Drücken der Druckknöpfe (54a, 55a) für die zwei Schalter aufzunehmen.

8. Gerät gemäß Anspruch 6, **dadurch gekennzeichnet, dass**
die zwei Schalter (54, 55) einen ersten Schalter (54), der an der Oberseite der Schalterbefestigungsoberfläche (52a) angeordnet ist und der dazu eingerichtet ist, eine häufig benutzte erste chirurgische Funktion aus der Mehrzahl von chirurgischen Funktionen auszuwählen, und einen zweiten Schalter (55) haben, der an der Unterseite der Schalterbefestigungsoberfläche (52a) angeordnet ist und der dazu eingerichtet ist, eine weitere zweite chirurgische Funktion aus der Mehrzahl von chirurgischen Funktionen auszuwählen.

9. Gerät gemäß Anspruch 8, **dadurch gekennzeichnet, dass**
die erste chirurgische Funktion eine Funktion zur gleichzeitigen Ausgabe einer Ultraschallbehandlungsausgabe und einer Hochfrequenzbehandlungsausgabe ist und
die zweite chirurgische Funktion eine Funktion zur unabhängigen Ausgabe der Hochfrequenzbehandlungsausgabe allein ist.

10. Gerät gemäß Anspruch 8, **dadurch gekennzeichnet, dass**
die erste chirurgische Funktion eine Funktion zur Ausgabe einer Ultraschallbehandlungsausgabe in einem maximalen Ausgabezustand ist und
die zweite chirurgische Funktion eine Funktion zur Ausgabe einer Ultraschallbehandlungsausgabe in einem vorgegebenen willkürlich festgesetzten Ausgabezustand ist, der niedriger als der maximale Ausgabezustand ist.

11. Gerät gemäß Anspruch 4, **dadurch gekennzeichnet, dass**
ein Fingerpolsterabschnitt (621, 622), der aus einem elastischen Material geformt ist, lösbar mit dem Daumeneinführringabschnitt (62) und/oder dem Ringabschnitt (61) zum Einführen mehrerer Finger verbunden ist.

12. Gerät gemäß Anspruch 4, **dadurch gekennzeichnet, dass**
das bewegliche Handgriffelement (49) einen Fingerhaken (601) in einem oberen Abschnitt des Daumeneinführringabschnitts (62) hat.

## Revendications

1. Appareil pour opération chirurgicale comprenant :
une partie d'insertion (5) qui contient une extrémité distale et une extrémité proximale et qui comprend un axe long et qui est configurée pour être insérée dans un corps ;
une partie de traitement (1A) qui est disposée au niveau de l'extrémité distale de la partie d'insertion (5) et qui possède une pluralité de fonctions chirurgicales sélectionnables ;
une partie de commande (4) disposée au niveau de l'extrémité proximale de la partie d'insertion (5), la partie de commande (4) présentant un côté avant et deux côtés latéraux ;
une pluralité de commutateurs (54, 55) qui sont prévus dans la partie de commande (4) et qui sont configurés pour sélectionner les fonctions chirurgicales ; et
une partie de renflement (501) qui est disposée entre les commutateurs (54, 55) et qui divise les commutateurs (54, 55) et qui est double en tant que partie de réception de doigts, dans laquelle la partie de commande (4) comporte, sur le côté avant de la partie de commande (4), une surface (52a) de fixation de commutateurs sur laquelle la pluralité de commutateurs (54, 55) sont fixés, et
la pluralité de commutateurs (54, 55) sont agencés dans un sens vertical de la surface (52a) de fixation de commutateurs,
**caractérisé en ce que** la partie de renflement (501) comprend une extension (502) qui s'étend de manière continue à partir de la surface (52a) de fixation de commutateurs de la partie de commande (4) sur au moins l'un des deux côtés latéraux de la partie de commande (4).

2. Appareil selon la revendication 1, **caractérisé en ce que**
la partie de renflement (501) est établie de sorte que la hauteur de projection de cette partie de renflement à partir de la surface (52a) de fixation de commutateurs est supérieure à la hauteur de projection de la pluralité de commutateurs (54, 55) à partir de la surface (52a) de fixation.

3. Appareil selon la revendication 1, **caractérisé en ce que**
la partie de commande (4) comporte un corps principal de cette partie de commande (4) et deux éléments de poignées (47, 49) pour actionner la partie de traitement,
les deux éléments de poignées (47, 49) comporte un élément de poignée fixe (47) fixé au corps principal de la partie de commande (4) et s'étendant sur un côté latéral de l'axe long, et un élément de poignée mobile (49) supporté pour pouvoir être ouvert/fermé par rapport à l'élément de poignée fixe (47), et
la surface (52a) de fixation est prévue au niveau d'une jonction entre le corps principal de la partie de commande (4) et l'élément de poignée fixe (47).

4. Appareil selon la revendication 3, **caractérisé en ce que**
l'élément de poignée mobile (49) comporte une partie annulaire (62) d'insertion de pouce qui permet l'insertion d'un pouce,
l'élément de poignée fixe (47) comporte une partie annulaire (61) d'insertion de plusieurs doigts qui permet l'insertion d'une pluralité de doigts exceptés le pouce et l'index,
la surface (52a) de fixation de commutateurs comporte une surface incurvée (506) incurvée le long d'une ligne de glissement (L1) sur laquelle l'index est mobile dans une condition dans laquelle le pouce est inséré dans la partie annulaire (62) d'insertion de pouce et la pluralité de doigts exceptés le pouce et l'index sont insérés dans la partie annulaire (61) d'insertion de plusieurs doigts.

5. Appareil selon la revendication 4, **caractérisé en ce que**
la partie de commande (4) est établie de sorte qu'un angle [alpha] entre une ligne tangente (L2) d'une surface avant de la partie annulaire (61) d'insertion de plusieurs doigts de l'élément de poignée fixe (47) et une ligne tangente (L3) d'une surface avant de la surface (52a) de fixation de commutateurs est supérieur à 90°.

6. Appareil selon la revendication 1, **caractérisé en ce que**
le corps principal de la partie de commande (4) comporte une unité de commutateurs (503), et la pluralité de commutateurs comprend deux commutateurs (54, 55) qui sont intégrés dans une unité, et un récepteur (504) d'unité concave sur lequel l'unité de commutateurs (503) est fixée,
l'unité de commutateurs (503) comporte des boutons-poussoirs (54a, 55a) pour les deux commutateurs, une carte flexible de circuit de câblage (503a) pour les deux commutateurs, et un élément de base flexible (503c) dans lequel la carte des circuit de câblage (503a) est mise en oeuvre dans des éléments élastiques isolants, et
l'élément de base (503c) est fixé au récepteur(504) d'unité de sorte que cet élément de base se courbe le long de la surface incurvée (506).

7. Appareil selon la revendication 6, **caractérisé en ce que**
le récepteur (504) d'unité comporte, dans des parties correspondant aux boutons-poussoirs (54a, 55a) pour les deux commutateurs, des parties de bossages (505a, 505b) qui sont configurées pour recevoir une force pour pousser les boutons-poussoirs (54a, 55a) pour les deux commutateurs.

8. Appareil selon la revendication 6, **caractérisé en ce que**
les deux commutateurs (54, 55) comportent un premier commutateur (54) qui est disposé sur le côté supérieur de la surface (52a) de fixation de commutateurs et qui est configuré pour sélectionner une première fonction chirurgicale fréquemment utilisée parmi la pluralité de fonctions chirurgicales, et un deuxième commutateur (55) qui est disposé sur le côté inférieur de la surface (52a) de fixation de commutateurs et qui est configuré pour sélectionner une autre deuxième fonction chirurgicale parmi la pluralité de fonctions chirurgicales.

9. Appareil selon la revendication 8, **caractérisé en ce que**
la première fonction chirurgicale est une fonction pour délivrer en sortie simultanément une sortie de traitement par ultrasons et une sortie de traitement haute fréquence, et
la deuxième fonction chirurgicale est une fonction pour délivrer en sortie indépendamment la sortie de traitement haute fréquence seule.

10. Appareil selon la revendication 8, **caractérisé en ce que**
la première fonction chirurgicale est une fonction pour délivrer en sortie une sortie de traitement par ultrasons dans un état de sortie maximal, et
la deuxième fonction chirurgicale est une fonction pour délivrer en sortie une sortie de traitement par ultrasons dans un état de sortie établi préétabli de manière arbitraire inférieur à l'état de sortie maximal.

11. Appareil selon la revendication 4, **caractérisé en ce que**
une partie (621, 622) de coussinet pour les doigts formée d'un matériau élastique est fixée de manière détachable sur au moins l'une parmi la partie annulaire (62) d'insertion de pouce et la partie annulaire (61) d'insertion de plusieurs doigts.

12. Appareil selon la revendication 4, **caractérisé en ce que**
l'élément de poignée mobile (49) comporte un crochet (601) pour doigt dans une partie supérieure de la partie annulaire (62) d'insertion de pouce.
